# EUROPEAN PATENT SPECIFICATION

(11) **EP 2 579 765 B1**
(45) Date of publication and mention of the grant of the patent: **07.08.2019**
(21) Application number: 11795292.9
(22) Date of filing: 13.06.2011
(51) Int. Cl.: A61B 1/005, A61B 10/02, A61B 5/107, A61M 19/00, A61M 25/01, A61M 5/00, A61B 5/00, A61B 17/34

(54) **ANATOMICAL-POSITIONING APPARATUS WITH AN EXPANDABLE DEVICE**
VORRICHTUNG FÜR ANATOMISCHE POSITIONIERUNG MIT EINER DEHNBAREN VORRICHTUNG
APPAREIL DE POSITIONNEMENT ANATOMIQUE AVEC UN DISPOSITIF EXPANSIBLE

(30) Priority: 13.06.2010 US 354225 P
(43) Date of publication of application: 17.04.2013
(73) Proprietor: Omeq Medical Ltd., M.P. Misgav 20174 (IL)
(72) Inventor: TSAMIR, Oded, 69412 Tel-Aviv (IL); MARGALIT, Lior, 62665 Tel-Aviv (IL)
(74) Representative: Pearl Cohen Zedek Latzer Baratz UK LLP
(86) International application number: PCT/IL2011/000465
(87) International publication number: WO 2011/158227

(56) References cited:
- WO-A2-2008/097609
- WO-A2-2008/097609
- US-A- 5 423 760
- US-A1- 2006 122 555
- US-A1- 2008 140 006
- US-A1- 2009 036 869
- US-A1- 2010 069 851
- US-A1- 2010 087 782
- US-B1- 6 669 674
- US-B2- 6 613 038

## Description

### FIELD OF THE INVENTION

This invention relates to invasive medical devices and more particularly, the present invention relates to anatomical-positioning medical devices for controlled advancement thorough multiple tissues of various types and cavities disposed there between, until reaching a target position. For example, an Epidural anesthesia device to provide controlled access to the Epidural space, while preventing puncturing the Dura mater.

### BACKGROUND OF THE INVENTION

In medical procedures, needle insertion and localization are issues of great importance. Misplacement of a needle tip or a trocar can harm essential tissues such as blood vessels, nerves or internal organs and lead to major complications. For example, during a procedure of Epidural anesthesia the needle tip should be placed inside the Epidural space (ES), to thereby facilitate administration of anesthetic agents. This has become a common and effective procedure for controlling pain during childbirth, major surgery, and chronic back pain.

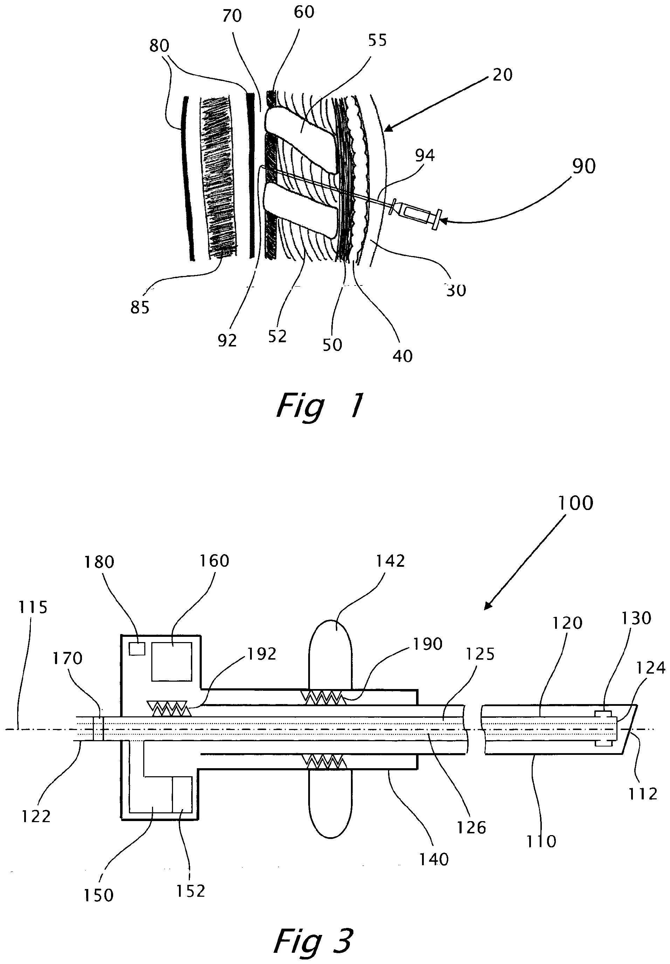

Reference is made to Fig. 1, an illustration of the final stage of an Epidural anesthesia procedure. The tip of a needle **92** of a device **90,** for administering a medication into Epidural space **70,** is disposed inside Epidural space **70,** after being inserted through skin **30** and advanced between Spinous process **55** and through the subcutaneous fat layer **40,** the Supraspinous Ligament **50,** the Interspinous Ligament **52** and the Ligamentum Flavum (LF) **60.**

Overshooting of the tip of the needle beyond the ES may puncture the Dura mater **80** causing a leak of the cerebral-spinal fluid (CSF) from spinal cord **85** into the ES, leading to severe headaches (post dural puncture headaches syndrome).

The majority of current injection techniques are "blind" techniques, mainly tactile based. For example, the main technique of Epidural block is based on the "loss of resistance technique" (LORT). In LORT, a fluid or air filled syringe is attached to a needle. While the needle is advanced through different layers in the insertion site, the physician taps on the syringe. Inside dense Ligament layers, the physician fills a strong resistance, but when crossing the LF **60** and entering the ES **70,** there is a substantial loss of resistance so that the fluid or air from the syringe can be easily pushed into the low-pressured ES **70,** thus signaling the physician to stop advancing needle **92.**

In practice, the LORT holds some major disadvantages. Because of the elastic properties of the LF **60,** the elastic fibers are pushed by the needle and are stretched into the ES **70.** For this reason, the rupture of these fibers takes place deep inside the ES **70** increasing the risk of an overshooting of the needle tip into the Dura mater **80.** Moreover, the resolution of the non-controlled advancement-increments of the needle-tip is very limited and differs extensively from one physician to another. Another disadvantage of LORT is the relatively high risk of a false loss of resistance, taking place for instance inside the LF **60** due to a small space between adjacent fibers.

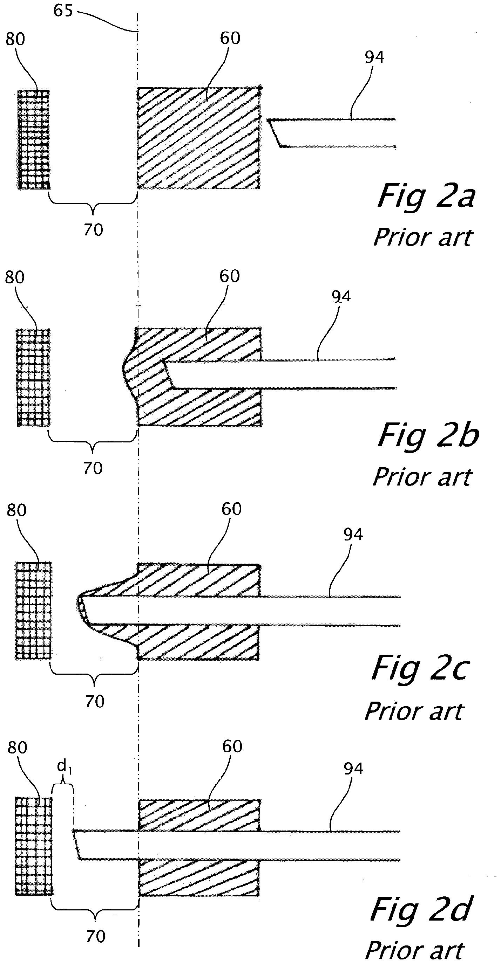

Figs. 2a-2d illustrate cross sectional views of the stages of a typical Epidural anesthesia procedure, including the penetration of Ligamentum Flavum **60** and including entering into Epidural space **70.** When needle **95** is advanced through Ligamentum Flavum **60,** the elastic fibers of Ligamentum Flavum **60** are stretched by pushing pressure exerted by needle **95** deep into Epidural space **70,** before entering Epidural space **70** (see Figs 2b and 2c). When the fibers reach a certain displacement, Ligamentum Flavum **60** ruptures and the needle penetrates into Epidural space **70,** as depicted in Fig. 2d, typically stopping a short distance (*d₁*) from Dura mater **80.** The displacement required for the fiber to rupture differs from one person to another due to physiologic variations in Ligamentum Flavum elasticity, thickness and other factors. However, using the prior art technique has an extensive risk of accidently puncturing the Dura mater due to overshooting of the needle.

US patent 5,188,594, given to Michael Zilberstein, provides a device and method for administering medicine into the Epidural space by first supplying air through a valve connected to a needle and provided with an inflatable element so that before the needle reaches the Epidural space the inflatable element is inflated. The needle is advanced and when the needle reaches the Epidural space **70** the inflatable element is deflated, which deflation is, typically, observed by the physician (or his/her stuff); medication is then administered through the valve and through the needle into the Epidural space.

PCT patent WO2008097609, given to Alexander Slocum et al, discloses apparatus for providing feedback regarding the material in which the tip of said apparatus is located as the tip is advanced into matter of varying resistances. The apparatus responds to a change in pressure, force, or other parameter such that when the tip reaches matter of a certain resistance, a change in the parameter is sensed. The apparatus provides a driving force to a penetrating medical device, such as a needle, when the apparatus tip encounters material of high resistance. When the apparatus tip encounters a low resistance material, no further driving force is applied to the apparatus. An inner core may be advanced into the low resistance material for deployment of a gas or a liquid as desired.

There is a need for and it would be advantageous to have an injection device that can overcome the disadvantages mentioned hereabove, including the disadvantages of the LORT, and is relatively a low cost solution.

The terms "Epidural anesthesia procedure" and "Epidural block procedure" are used herein interchangeably.

### SUMMARY OF THE INVENTION

By way of introduction, the principal intentions of the present invention include providing an anatomical-positioning apparatus, such as an injection device, that facilitates acquiring mechanical data from a mammalian tissue by an expandable device, while the expandable device exerts pressure onto portions of said mammalian tissue. Thereby facilitating to determine the type of a mammalian tissue, as well as determining transition between different mammalian tissues and cavities. An aspect of the present invention is to provide safe methods for advancing an introducer, typically a sharp introducer, from an elastic tissue to a soft tissue, such as from the Ligamentum Flavum to the Epidural space.

According to the teachings of the present invention there is provided an anatomical-positioning apparatus for acquiring mechanical data from a mammalian tissue to facilitate determining a type of the mammalian tissue and determining transitions between different mammalian tissues and cavities. The anatomical-positioning apparatus includes a cannula having a tip at the distal section thereof and an external circumference that is proximal to the tip, an expandable device, securely attached to the tip or on the external circumference of the cannula; the expandable device comprising at least one of: a balloon, a membrane, a diaphragm, a spring and a flexible device, wherein the expandable device has a contracted form size when in a contracted state, and an expanded form size of preconfigured volume when in an expanded state, wherein the expanded form size is substantially larger than the contracted form size. The anatomical-positioning apparatus further includes an introducer, having a longitudinal axis and a distal end, the introducer arranged to penetrate and advance through the mammalian tissue and to facilitate an introduction of the expandable device into the mammalian tissue. The anatomical-positioning apparatus further includes an expanding mechanism arranged to controllably expand and contract the expandable device within the mammalian tissue to displace the tissue; an advancement mechanism arranged to incrementally and controllably advance at least one of: the introducer, the cannula, the expandable device, and a combination thereof; and a sensor arranged to measure physical parameters associated with the expandable device and correlated with a mechanical response of the tissue to the displacement by the expandable device. The anatomical-positioning apparatus also includes a processor arranged to record and analyse the measurements and determine therefrom the type of the mammalian tissue and the transitions between different mammalian tissues and cavities; wherein the advancement mechanism and the expanding mechanism are configured to: introduce the expandable device into the mammalian tissue, the introducer being advanced incrementally through the mammalian tissue either together with the cannula and the expandable device, or incrementally with respect to the cannula and the expandable device. The advancement mechanism and the expanding mechanism are further configured to: expanding the expandable device by the expanding mechanism outside the tip of the cannula from said contracted form sized to said expanded form size of preconfigured volume to exert pressure onto portions of the mammalian tissue to displace the mammalian tissue from an undistorted position to a distorted position; and, said sensor is arranged to measure one or more measurements selected from a group of measurements consisting of the pressure inside the expandable device, an external force applied on the expandable device by the mammalian tissue, and the spatial pressure or spatial force applied on the expandable device by the mammalian tissue, while the expandable device exerts pressure onto portions of the mammalian tissue.

The sensed data is acquired while the expandable device exerts pressure onto portions of the mammalian tissue, to thereby facilitating determining the type of tissue that is in contact with the expandable device and to determine a transition between different tissues and cavities. The sensor includes one or more sensing devices selected from the group of devices consisting of a pressure sensor, strain gauge, force sensor, tactile sensor, displacement sensor, volume sensor, flow sensor and a piezoelectric transducer. Optionally, the sensing device includes an array of sensors disposed on the expandable device.

Typically, the sensed data is indicated by one or more indication devices selected from the group of devices consisting of a display, a gage, a light indicator, an audio indicator and a tactile indicator.

Optionally, the sensed data includes a measurement of the advancement of the cannula against the mammalian tissue, and wherein the displacement of the expandable device, along the longitudinal axis, is inferred from the sensed data.

Optionally, the sensed data includes a one-dimensional measurement of the displacement of the expandable device, during the expanding against the mammalian tissue or contracting away from the mammalian tissue.

According to the invention, the sensed data facilitates determining one or more measurements selected from a group of measurements consisting of the volume-pressure work performed by the expandable device, a volume-pressure profile of the expandable device, the work being done by the expandable device and the force-displacement profile of the expandable device, while the expandable device exerts pressure onto portions of the mammalian tissue.

The expandable device is securely attached to the tip of the cannula or on the external circumference of the cannula, proximal to the tip of the cannula and when the expandable device expands from the contracted state to the expanded form size, the expandable device is disposed outside the introducer, and a blunt contact surface is formed at the external surface of the expanded expandable device.

The expandable device protects the mammalian tissue or an anatomical structure ahead of the mammalian tissue, from being punctured by the introducer of the expandable device. The cannula protects the expandable device from being punctured by the introducer. Typically, the expandable device expands and contracts under fluid or gas pressure provided by the expanding-mechanism through the cannula.

In variations of the present invention, the introducer is an openable introducer, having an openable sharp tip, wherein the openable sharp tip of the openable introducer has a closed state and an open state, and wherein the expandable device is trapped inside the openable sharp tip, the openable sharp tip being in a closed state. Preferably, the expandable device facilitates the openable sharp tip to remain in the closed state, when the expandable device is loaded by a preconfigured expanding force and the sharp tip is situated in a high density tissue. The expandable device facilitates the opening of the openable sharp tip, when the expandable device is loaded be a preconfigured expanding force and the openable sharp tip enters a low density tissue or cavity. The closed state facilitates advancement of the openable introducer through the mammalian tissue, while the open state prevents advancement of the openable introducer through the mammalian tissue. It should be noted that the expandable device also protects soft tissues ahead of expandable device.

Optionally, the introducer is selected from the group consisting of a veress needle, an epidural needle, a biopsy needle, a trocar, a cannula, a catheter, a Tuohy type needle and a surgical instrument.

Optionally, the expandable device includes one or more mechanical devices selected from a group of devices consisting of a spring and a balloon enclosed in a conical element, wherein the conical element protects the balloon from being punctured.

Optionally, the cannula and the expandable device is introduced on-demand and can be withdrawn during a procedure in order to enable utilization of other tools inside the mammalian tissue.

Optionally, the cannula includes a double lumen, wherein a first lumen facilitates a pathway for the fluid or gas into the expandable device, and wherein a second lumen facilitates a pathway for one or more devices selected from a group of devices consisting of a thin needle, a catheter, an optical fiber, an electrode and a surgical instrument.

Optionally, the cannula includes a single lumen facilitating a pathway for the fluid or gas into the expandable device, and wherein the single lumen facilitating a pathway for an optical fiber or a miniature camera, to thereby facilitate visualization of the mammalian tissue through the expandable device, the expandable device being in contact with the mammalian tissue.

Optionally, the advancement mechanism is selected from the group of devices consisting of a motor based device, hydraulic device, gear based device and a screw based device. According to the invention, the introducer is advanced incrementally together with the cannula and the expandable device or the introducer is advanced incrementally with respect to the cannula and the expandable device. The sensed data is acquired while the expandable device is expanding, contracting or in a steady expansion level.

### BRIEF DESCRIPTION OF THE DRAWINGS

The present invention will become fully understood from the detailed description given herein below and the accompanying drawings, which are given by way of illustration and example only and thus not limitative of the present invention, and wherein:
Fig. 1 illustrates the Epidural space and surrounding anatomical structures with a needle properly inserted into the Epidural space;
Figs. 2a-2d (prior art) illustrate cross sectional views of the stages of a typical Epidural anesthesia procedure, including penetration of the Ligamentum Flavum and including entering into the Epidural space;
Fig. 3 is a schematic illustration of an anatomical-positioning apparatus, according to embodiments of the present invention.
Figs. 4a-4e illustrate cross sectional views of the distal portion of an anatomical-positioning apparatus as shown in Fig. 3, showing the stages of an Epidural anesthesia procedure, including expanding an expandable device inside the Ligamentum Flavum and inside Epidural space;
Fig. 5 illustrates another embodiment of an anatomical-positioning apparatus, according to embodiments of the present invention, having a spring and a conical element, shown in a closed state;
Fig. 6 illustrates the anatomical-positioning apparatus shown in Fig. 5, the conical element being in an opened state;
Fig. 7 is a graph that describes the resistance forces being measured when advancing a plastic tube with a surface area of ∼4 mm² against Ligamentum and against Fat.
Fig. 8 is a graph that describes the resistance forces being measured when advancing a "Tuohy" 18G needle against Ligamentum and against Fat.
Fig. 9 is a schematic flow chart showing an exemplary method of determining a tissue type, as well as transition between different tissues and cavities, using an anatomical-positioning apparatus, according to embodiments of the present invention.
Figs. 10a-10e illustrate cross sectional views of the distal portion of an anatomical-positioning apparatus, as shown in Figs. 5 and 6, showing the stages of a spring-back method of safe penetration of the Ligamentum Flavum and entering into the Epidural space, during an Epidural anesthesia procedure.
Fig. 11 is a schematic flow chart showing an exemplary spring-back method as illustrated in Figs. 10a-10e.
Figs. 12a-12d illustrate side views of the distal portion of an anatomical-positioning apparatus, according to variations of the present invention, having a thin-needle being introduced through a cannula for facilitating a spring-back method.
Figs. 13a-13g illustrate cross sectional views of the distal portion of an anatomical-positioning apparatus, as shown in Figs. 12a-12d, showing the stages of a spring-back method of safe penetration of the Ligamentum Flavum and entering into the Epidural space, during an Epidural anesthesia procedure.
Fig. 14 is a schematic flow chart showing an exemplary spring-back method as illustrated- - in Figs. 13a-13g.
Fig. 15 illustrates the distal section of an anatomical-positioning apparatus, according to variations of the present invention, wherein the introducer includes an openable tip and wherein the openable tip is shown in a closed state.
Fig. 16a illustrates a cross sectional view of the distal portion of the anatomical-positioning apparatus shown in Fig. 15, wherein the introducer penetrates an mammalian tissue, such as the Ligamentum Flavum, and wherein the openable tip is shown in a closed state.
Fig. 16b illustrates a cross sectional view of the distal portion of the anatomical-positioning apparatus shown in Fig. 15, wherein the introducer penetrates a mammalian tissue, such as the Ligamentum Flavum, and wherein the openable tip is shown in an opened state.
Fig. 17 is a schematic flow chart showing an exemplary method safely penetrating the Ligamentum Flavum and entering Epidural space using the anatomical positioning apparatus illustrated in Figs. 15-16b.
Fig. 18a-18b illustrate side views of an anatomical-positioning apparatus, according to variations of the present invention, having a guide-wire attached to the expandable device for measuring the displacement of the expandable device.

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided ,so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The methods and examples provided herein are illustrative only and not intended to be limiting.

Referring back to Fig. 1 illustrating Epidural space **70 and** surrounding anatomical structures with a tip **92** of needle **90** properly inserted into Epidural space **70.**

Referring is also to Fig. 3, showing a schematic illustration of an exemplary anatomical-positioning apparatus **100,** according to preferred embodiments of the present invention. Anatomical-positioning apparatus **100** includes a housing **140** that secures both a sheath-shaped introducer **110,** having a longitudinal axis **115,** and an expandable device **130,** which expandable device **130** is securely attached to a cannula **120.** Anatomical-positioning apparatus **100** further includes a cannula **120,** having an opened proximal end **122,** a distal end **124,** and two lumens, wherein a first lumen **125** facilitates the insertion and evacuation of gas or fluid into expandable device **130,** and a second lumen **126** facilitates the insertion of various elongated devices such as a catheter. Expandable device **130** is securely attached to the external circumference of cannula **120,** proximal to tip **124** of cannula **120.**

In variations of the present invention, cannula **120** has an elongated hollow body (single lumen) facilitating the insertion and evacuation of gas or fluid into expandable device **130,** wherein expandable device **130** is securely attached to distal end **124** (tip) of cannula **120.** The elongated hollow body of cannula **120** can also provide a pathway for an optical fiber or a miniature camera, to thereby facilitate visualization of a mammalian tissue or cavity through expandable device **130,** when expandable device **130** is expanded.

In embodiments, introducer **110** can be a Veress needle, an Epidural needle, a biopsy needle, a trocar, a cannula, a catheter, a Tuohy type needle, a surgical instrument or any other sharp object facilitated to be inserted into at least one mammalian tissue.

It should be noted that the present invention will be described mostly in terms of an anatomical-positioning apparatus for performing a procedure of Epidural anesthesia, including determining the type of one or all tissues the introducer is advancing through, including Interspinous Ligament **52,** Ligamentum Flavum **60** and inside Epidural space **70,** as well as indicating transition between tissues and cavities. But the present invention is not limited to anatomical-positioning apparatus for performing a procedure of Epidural anesthesia, including determining the type of one or all tissues the introducer is advancing through and indicating transition between tissues and cavities. The present invention includes all anatomical-positioning apparatuses for determining the type of one or more tissues, the introducer is advancing through, as well as indicating transition between different tissues and cavities.

Distal end **112** of introducer **110** is typically, with no limitation, a sharp end. Cannula **120** can be made of any biocompatible material, preferably hard plastic or stainless still. Expandable device **130** can be expanded to a preconfigured volume or shape and have a preconfigured displacement along longitudinal axis **115** of introducer **110** using air or fluid passing through first lumen **125** of cannula **120** and into expandable device **130.**

Alternatively, the expandable device can be made of a shape memory alloy, such as NiTi alloy, so that under certain conditions (for instance, heat) it expands. Optionally, the expandable device can be expanded using a mechanical mechanism that is based on springs or any other mechanical element. In one preferred embodiment of the present invention, without limiting the scope of this invention, expandable device **130** is an inflatable balloon that can be made of any elastic material known in the art, such as polyurethane, flexible PVC, PET, nylon, nylon elastomers and thermoplastic elastomers.

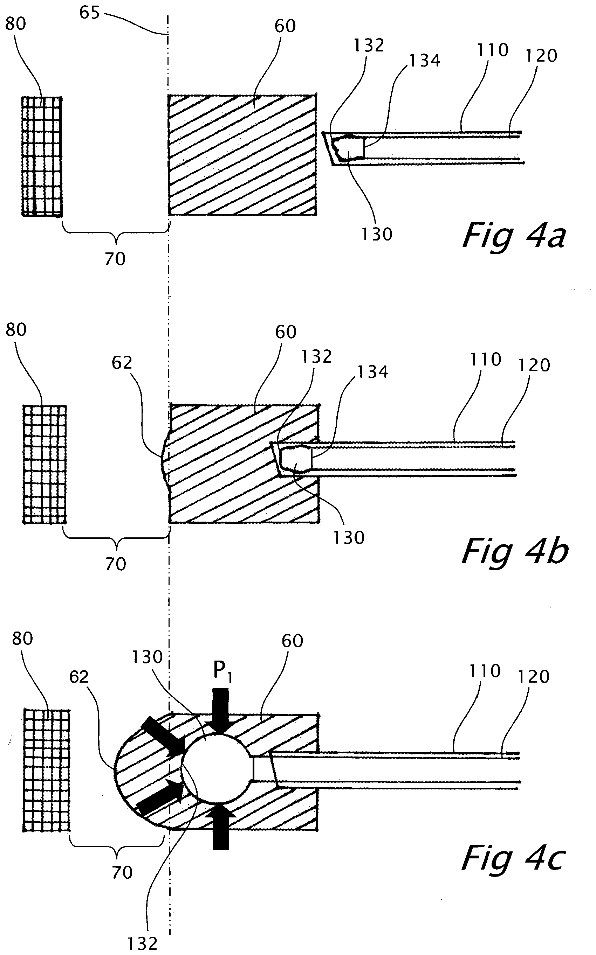

Reference is now made to Figs. 4a-4e, illustrating cross sectional views of the- - distal portion of anatomical-positioning apparatus **100,** showing the stages of an Epidural anesthesia procedure, including the penetration of Ligamentum Flavum **60** and including entering into Epidural space **70.** Fig. 4a illustrates expandable device **130** as a balloon, being in a contracted state, while Fig. 4c illustrates expandable device **130** as a balloon, being in an expanded state. Proximal end **134** of expandable device **130** is securely attached to distal end **124** (tip) of cannula **120.**

In the contracted state, balloon **130** is completely deflated, and is protectively disposed inside introducer **110.** Balloon **130** is kept deflated by mechanical biasing force or by maintaining partial vacuum-pressure inside balloon **130.**

In the expanded state or in a partially expanded state, balloon **130** is popped out of introducer **110,** such that introducer **110** does not damage balloon **130.** Balloon **130** is kept inflated by providing controlled gas or fluid pressure into balloon **130.** When balloon **130** is inflated, distal end **132** of expandable device **130** forms a blunt surface.

Referring back to Fig. 3, Anatomical-positioning apparatus **100** further includes an expanding-mechanism **150** for expanding and contracting expandable device **130.** When expandable device **130** is a balloon, the expanding-mechanism, typically, includes a pump (not shown) for controllably pump gas (air) or fluid into and out of balloon **130.** Expanding-mechanism **150** may include a container **152** for holding gas or fluid.

Anatomical-positioning apparatus **100** further includes a sensor **170** for measuring physical parameters associated with expandable device **130,** while expandable device **130** exerts pressure onto portions of the mammalian tissue being operationally pressed by expandable device **130.** The measuring of the physical parameters, associated with expandable device **130,** facilitates determining the type of tissue that is in contact with a blunt external surface, typically distal surface **132** of balloon **130,** as well as determining transition between tissues and cavities.

The measuring of the physical parameters, associated with expandable device **130,** may take place when expandable device **130** expands, when expandable device **130** contracts, or when expandable device **130** is already expanded and in a steady state.

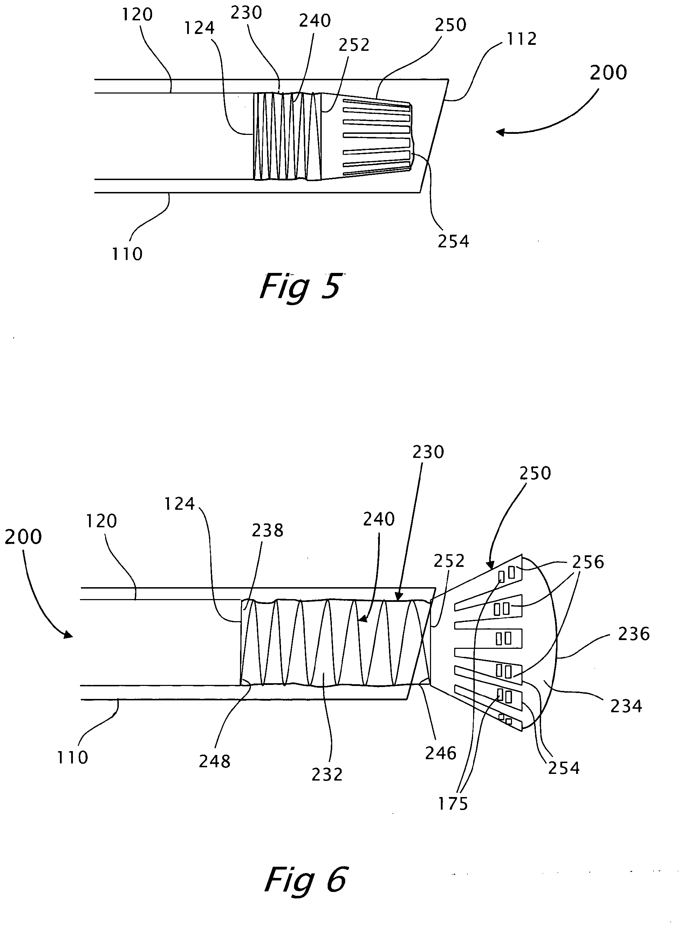

Reference is now made to Figs. 5-6, illustrating anatomical-positioning apparatus **200** including another embodiment of the expandable device, according to variations of the present invention.

As shown in Figs. 5 and 6, expandable device 230, such as balloon **230,** includes-having a proximal section **232** encapsulated inside a biasing element such as a helical spring **240** and a conical element **250** that are securely interconnected. When balloon **230** is inflated to a preconfigured volume, as depicted in Fig. 6, proximal section **232** of balloon **230,** having a diameter smaller than the internal diameter of introducer **110,** and distal section **234,** having a diameter that is typically, with no limitation, 1.1-4 times larger than the internal diameter of introducer **110,** and approximately 1.5-2 times larger, in the best mode.

Proximal end **238** of proximal section **232** of balloon **230** is securely attached to proximal end **248** of spring **240,** both of which are securely attached to distal end **124** (tip) of cannula **120.** The proximal end of distal end section **234** of balloon **230** is disposed at proximal end **252** of conical element **250,** wherein conical element **250** has a proximal end **252** and a distal end **254,** and wherein proximal end **252** of conical element **250** is securely attached to distal end **246** of spring **240.** Proximal end **252** of conical element **250** has a substantially constant diameter that fits inside the internal diameter of introducer **110.** In the collapsed state of expandable device **230,** distal end **254** of conical element **250** has a smaller or equal diameter to the diameter of proximal end **252** of conical element **250.** When expandable device **230** is expanded, distal end **254** of conical element **250** has a larger diameter than the internal diameter of introducer **110.**

As depicted in an exemplary embodiment in Fig. 6, conical element **250** includes on the distal section of conical element **250,** typically, the majority of the distal section of conical element **250,** a preconfigured number of elastic flaps **256** that are separated by a slot cut into the distal section of conical element **250.** Flaps **256** can operatively hingedly bend or deflect outwards as a result of pressure being built inside distal section **234** of balloon **230,** thus enabling distal section **234** of balloon **230** to expand to the preconfigured volume, distally outside of the typically sharp tip of introducer **110.**

Preferably, gas (preferably air) or fluid is injected through cannula **120** and into balloon **230,** thereby inflating balloon **230.** Hence, proximal section **232** of balloon **230** stretches spring **240** distally, towards distal end **112** of introducer **110,** and outside of distal end **112** of introducer **110.** As fluid pressure continues to build up, balloon further inflates to an extent that forces flaps **256** of conical element **250** to open, thus facilitating distal section **234** of balloon **230** to reach the preconfigured volume and surface area. Flaps **256** of conical element **250** protect distal section **234** of balloon **230** from being punctured by tip **112** of introducer **110.**

Upon deflation of balloon **230,** elastic flaps **256** apply the force, stored while being inflated, against distal section **234** of balloon **230,** thus collapsing distal section **234** of balloon **230** back into the smaller diameter of the contracted state. As the gas or fluid pressure inside balloon **230** continues to drop, balloon **230** further deflates and spring **240** returns to the unbiased position. Thereby, expandable device **230** is back inside introducer **110,** proximal to distal end **112,** as depicted in Fig. 5.

When the expandable device is expandable device **230,** the expanding-mechanism, typically, includes a pump (not shown) for controllably pump gas (air) or fluid into and out of balloon **230,** wherein the operation of the pump is synchronized with biasing element **240** and flaps **256.**

An aspect of the present invention is to facilitate determining the tissue type, as well as transition between tissues and cavities. The following describes a method describes the steps of an Epidural anesthesia procedure, by way of example, without limiting the scope of this invention. During Epidural anesthesia procedure, a physician performs procedural steps, in order to differentiate between Ligamentum Flavum **60** and Epidural space **70,** wherein a principle intention of the Epidural block procedure is to be able to stop the advancement of introducer **110** upon entering Epidural space **70.**

In an embodiment of the present invention, during the inflation of balloon **130,** a measurement that reflects the mechanical resistance of the tissue to the inflation of balloon **130,** is taken, using selected sensors **170** (see Fig. 3) disposed in pre configured location, within anatomical-positioning apparatus **100.** Such sensors can be force sensors, pressure sensors, displacement sensors, strain gauges, tactile sensors, volume sensors, flow sensors, piezoelectric sensors and any other sensors known in the art. The data collected by sensors **170** are sensed while expandable device **130** exerts pressure onto portions of the mammalian tissue. The sensed data is of physical parameters associated with expandable device **130,** and reflects mechanical properties of the tissue in which tissue expandable device **130** is disposed while obtaining the sensed data.

The measured physical parameters, associated with expandable device **130,** may include the pressure inside balloon **130,** the instantaneous volume of balloon **130,** the displacement of expandable device **130,** the external force applied on the expandable device by the mammalian tissue, spatial force or spatial pressure applied on expandable device **130** or any other measurement known in the art. Other data can be concluded by processing the sensed data using a processor **160,** such as the volume-pressure work -performed-by-expandable device **130**, volume pressure profile-of-expandable-device **130,** work performed by expandable device **130** and force-displacement profile of expandable device **130,** while expandable device exerts pressure onto portions of a mammalian tissue, For example, a pressure sensor can measure the pressure that builds up inside balloon **130** for a given volume. According to the measurement, the type of tissue being pushed by balloon **130** is determined and a decision is made whether to advance introducer **110** by another increment or whether to stop introducer **110** from further advancing, due to entrance into Epidural space **70.**

The measurement may be continuous during the expansion, the contraction or the steady expanded state of expandable device **130,** thus providing a unique set of measurements for a specific tissue. For instance, a measurement of the external force applied by a mammalian tissue against an expanded expandable device **130** as a function of the displacement of expandable device **130,** may provide a curve that is typical for a certain type of tissue, for example an elastic tissue, and is substantially different from a non-elastic tissue.

The measurement can be of spatial force or spatial pressure applied on expandable device **130** by a mammalian tissue. In one embodiment, tactile sensors **175** disposed on expandable device **130** provide spatial data reflecting the mechanical resistance of the tissue being pressed by expandable device **130** in various locations along the circumference of expandable device **130.** Such measurements can facilitate determining the direction of the fibers of the mammalian tissue being pressed by the expandable device **130.** In addition, such measurements can facilitate determining the existence and direction of a hard tissue (such as a spinal process) that is in the vicinity of the mammalian tissue being pressed by the expandable device **130.**

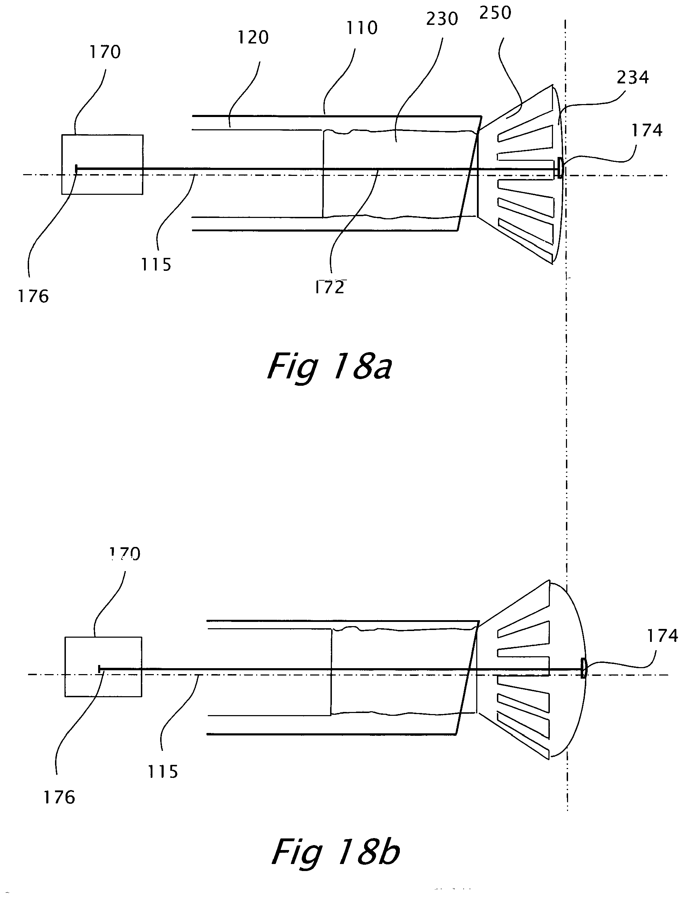

Fig 18a-18b illustrate an embodiment of the present invention, wherein a distal tip **174** of a guide-wire **172** is attached to distal end **234** of balloon **230,** and proximal tip **176** of guide-wire **172** is located near a displacement sensor **170.** Upon expansion of balloon **230,** distal end **234** of balloon **230** advances forward along longitudinal axis **115,** thereby facilitating the advancement of guide-wire **172** along longitudinal axis **115.** Displacement sensor **170,** for example a photoelectric sensor, measures the displacement of proximal end **176** of guide-wire **172,** thereby facilitating determining the displacement of balloon **230** while exerting pressure on portions of a mammalian tissue.

The main advantage of the method for determining tissue type and transition between tissues and cavities by an expandable device is in actively acquiring data regarding a mammalian tissue, by deforming the tissue, in order to determine mechanical properties, such as elasticity, that can otherwise be hard to discover by a passive measurement. Moreover, the larger surface area of distal end **132** of balloon **130,** compared the to the tip surface area of tip **112** of introducer **110,** finds an expression in that the mechanical resistance of elastic tissues is being amplified by the larger surface area of distal end **132,** whereas the mechanical resistance of loose tissues does not change significantly. Thus, it is possible to differentiate between tissues types, although there are variations in population.

In addition, when measuring forces that develop against an introducer **110,** friction forces operating on the body of introducer **110** have major influence on the measurement. However, when measuring forces against an expandable device **130,** the friction forces operating on the body of introducer **110** have no effect on the measurement. Therefore, a measurement of mechanical resistance against an expandable device is less biased than a measurement against introducer **110.**

One more advantage is in the ability to perform multiple measurement by expandable device **130** whether in a specific anatomical location or in a series of locations along the path of tip **112** of introducer **110.**

Indicator **180** indicates the type of tissue being in contact with expandable device **130,** as well as possible transition between different tissues and cavities. Such an indication can be visual, for example using a display showing a curve of force versus displacement of expandable device, light indication, acoustic indication, for instance when expandable device reaches Epidural space, a gage and a tactile indication, for example a vibration that is indicative to the extent of tissue elasticity.

Referring back to Figs. 4a-4e, cross sectional views of the distal portion of anatomical-positioning apparatus **100** are illustrated, showing the stages of an Epidural anesthesia procedure, including the penetration of Ligamentum Flavum **60** and including entering into Epidural space **70.** In fig. 4a, Ligamentum Flavum **60** is shown in an undistorted position, as introducer **110** has not touched Ligamentum Flavum **60** as yet. In fig. 4b, introducer **110** has started to penetrate Ligamentum Flavum **60** and thereby, Ligamentum Flavum **60** is not in an undistorted position, the elasticity of the fibers of Ligamentum Flavum **60** cause Ligamentum Flavum **60** to bend into Epidural space **70** (see concavity **62** in Figs. 4b and 4c).

As the operator does not see and thereby does not know that introducer **110** has penetrated Ligamentum Flavum **60,** the operator activates expanding mechanism **150** to thereby expand balloon **130.** The elasticity of Ligamentum Flavum **60** responds to expanded balloon **130,** disposed inside Ligamentum Flavum **60,** to set a pressure level *P₁* inside balloon **130.** Knowing, for example the quantity of gas/fluid inserted by expanding mechanism **150,** processor **160** analyzes measured pressure *P₁*, to thereby provide the type of tissue balloon **130** is disposed in. In the described example, processor **160** activates indicator **180** to indicate to the operator that balloon **130** is disposed inside Ligamentum Flavum **60.**

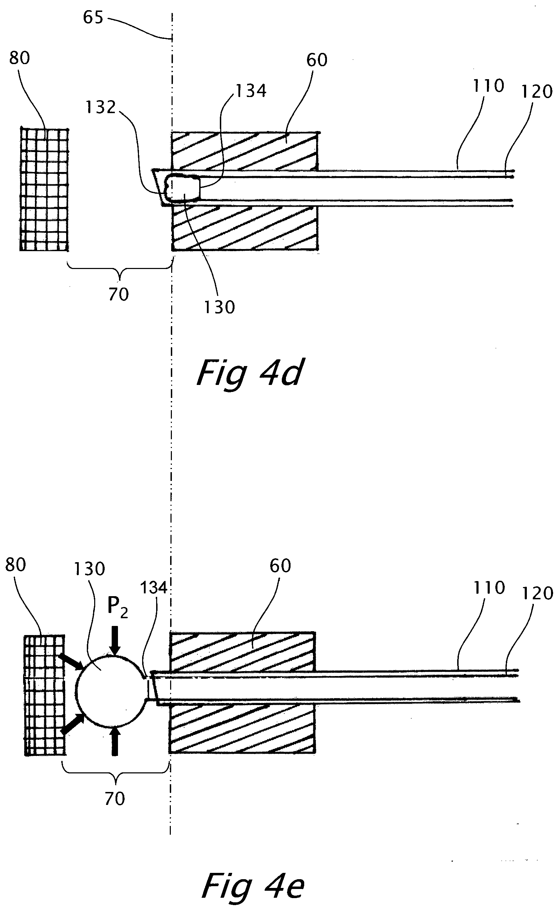

Typically, the operator of anatomical-positioning apparatus **100** activates expanding mechanism **150** to bring back expand balloon **130** to contracted state. Alternatively, balloon **130** automatically contracts back to a contracted state. Then, the operator activates an incremental mechanism for controllably advancing both introducer **110** and cannula **120** further towards Epidural space **70.** The operator may activate an introducer-incremental-advancing-mechanism **190** (see Fig. 3) and/or a cannula-incremental-advancing-mechanism **192.** As the incremental advancing is repeatably performed by the operator, introducer **110** ruptures Ligamentum Flavum **60** penetrates into Epidural space **70,** as depicted in Fig. 4d. Introducer-incremental-advancing-mechanism and cannula-incremental-advancing-mechanism **192** are shown in Fig. 3 schematically. The incremental advancing mechanism may be embodied in ways know in the art, such as a screw based mechanism having threads with a preconfigured pitch of 0.5-2 mm, a step motor etc.

Since the anatomical-positioning apparatus **100** is held by the operator's hands and is inserted into a living body, movements of both the operator and the patient may interfere with positioning of tip **112** of introducer **110** inside a mammalian tissue. Therefore, when using the incremental advancing mechanism, the anatomical-positioning apparatus **100** may be stabilized by securing housing **140** of apparatus **100** against the entrance site of introducer **110,** for instance, the back of a patient.

When the operator activates expanding mechanism **150** to thereby expand balloon **130,** the fat tissue inside Epidural space **70** responds to expanded balloon **130** to set a pressure level *P₂* (see Fig. 4e) inside balloon **130.** Knowing, for example the quantity of gas/fluid inserted by expanding mechanism **150,** processor **160** analyzes measured pressure *P₂*, to thereby provide the type of tissue balloon **130** is disposed in. In the described example, processor **160** activates indicator **180** to indicate to the operator that balloon **130** is disposed inside Epidural space **70.**

### An exemplary experiment:

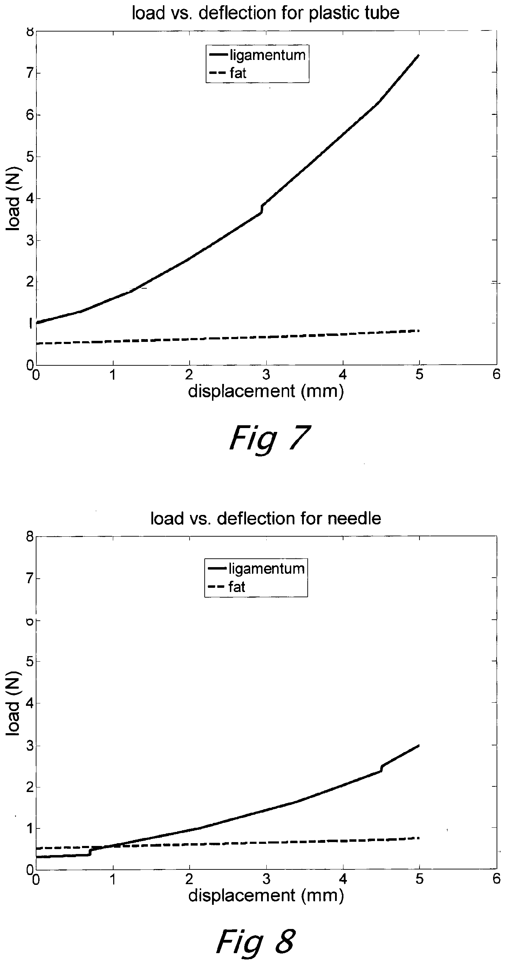

In Fig. 7, the graph describes the resistance forces being measured when advancing a plastic tube with a surface area of ∼4 mm² against a Ligamentum and against fat (wherein the fat simulates the Epidural fat tissue in Epidural space **70**). The plastic tube simulates the blunt surface of balloon **130,** having a larger surface area than a needle tip. In Fig. 8, the graph describes the resistance forces being measured when advancing a "Tuohy" 18G needle against a Ligamentum and against fat. The graphs demonstrate a substantial bigger resistance forces measured in the Ligamentum, when advancing a plastic tube, compared to advancing the needle. However, no significant difference in the resistance forces was measured when advancing the plastic tube or the needle against fat tissue. Therefore, the method of measuring forces against an expandable device holds some major advantages and facilitates setting a threshold for differentiating between tissues with different mechanical properties.

Therefore, it is clear that displacing an elastic tissue increases its mechanical resistance, thereby increasing the pressure measured inside a balloon **130,** while the resistance of a non elastic and low density tissue remains substantially unchanged under similar displacement. Hence, active measurement of the resistance of a tissue, while expanding or contracting balloon **130** provides data that facilitate determining the type of tissue balloon **130** is situated in.

In variation of the present invention, a ratio between the previous measurement and current measurement is calculated. If the ratio is smaller than a preconfigured threshold value, it is determined that balloon **130** is still inside the Ligamentum Flavum **60,** and introducer **110** is incrementally advanced. If the ratio is bigger than the preconfigured value, it means that introducer **110** is inside Epidural space **70.** Balloon **130** is deflated and cannula **120** is removed from anatomical-positioning apparatus **100** to enable threading of a catheter through introducer **110** to administrate anesthetics.

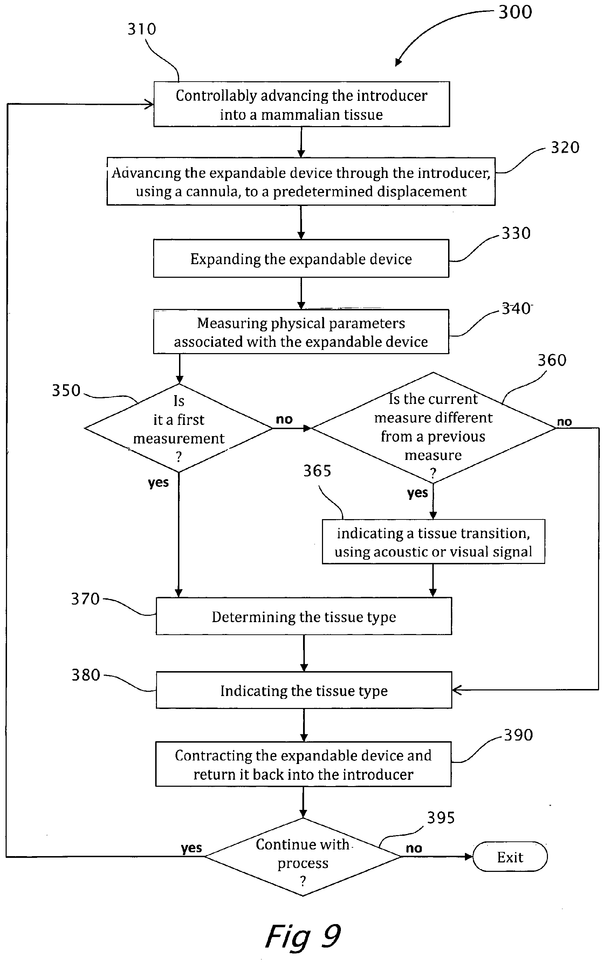

Reference is now made to Fig. 9, a schematic flow chart, showing an exemplary method **300** of determining the tissue type and transition between tissues and cavities, according to embodiments of the present invention. Method **300** begins by administrating introducer **110** of anatomical-positioning apparatus **100** into a mammalian tissue. Method **300** proceeds with the following steps:
**Step 310:** controllably advancing introducer **110** into a mammalian tissue.
   The operator of anatomical-positioning apparatus **100** controllably advances introducer **110** inside a mammalian tissue.
**Step 320**: advancing expandable device **130** through introducer **110,** using cannula **120,** to a predetermined displacement.
   The operator advances expandable device **130** through introducer **110,** using cannula **120,** to a predetermined displacement. Preferably, the operator uses a cannula-incremental-advancing-mechanism **192,** to controllably advance expandable device **130.**
**Step 330:** expanding expandable device **130.**
   Expandable device **130** is popped out of introducer **110** and expanded, typically by pressurized flow of gas (typically, air) or fluid, performed by expanding mechanism **150.**
**Step 340:** measuring physical parameters associated with expandable device **130.**
   Typically, the pressure inside expandable device **130** is measured as a function of the volume of gas/fluid flown into expandable device **130,** and/or the external force applied on expandable device as a function of the displacement of expandable device, and/or any other physical feature being measured during expansion, contraction or expanded steady state of expandable device **130.**
**Step 350:** check if it is the first measuring for the current tissue type.
   If it is the first measuring for the current tissue type, go to step **370.**
   Else, preferably, compare current measurement with a previous measurement, typically, the last measurement.
**Step 360:** check, preferably by processor **160,** if the current measurement differs from a previous measure.
   If the current measurement is substantially the same (within a preconfigured threshold value) as the previous measurement, go to step **380.**
   Else, it is likely that the current measurement was measured in a different tissue type, compared to the tissue type in which the previous measurement was taken.
**Step 365:** indicating a tissue transition.
   Indicator **180** indicates a tissue transition event, using an acoustic, visual or any other indicatory signal.
**Step 370:** determining the tissue type.
   Determine, preferably by processor **160**, the tissue type, for example, by comparing the measured physical parameters to a database.
**Step 380:** indicating the tissue type.
   Indicator **180** indicates a tissue type to the operator.
**Step 390:** contracting expandable device **130.**
   Expandable device **130** is contracted by expanding mechanism **150,** typically by evacuating the gas or fluid from within expandable device **130,** and thereby, the contracted expandable device **130** also returns back into introducer **110.**
**Step 395:** check if procedure is complete.
   If the operator requests the termination of the process, terminate process.
   Else, go to step **310.**

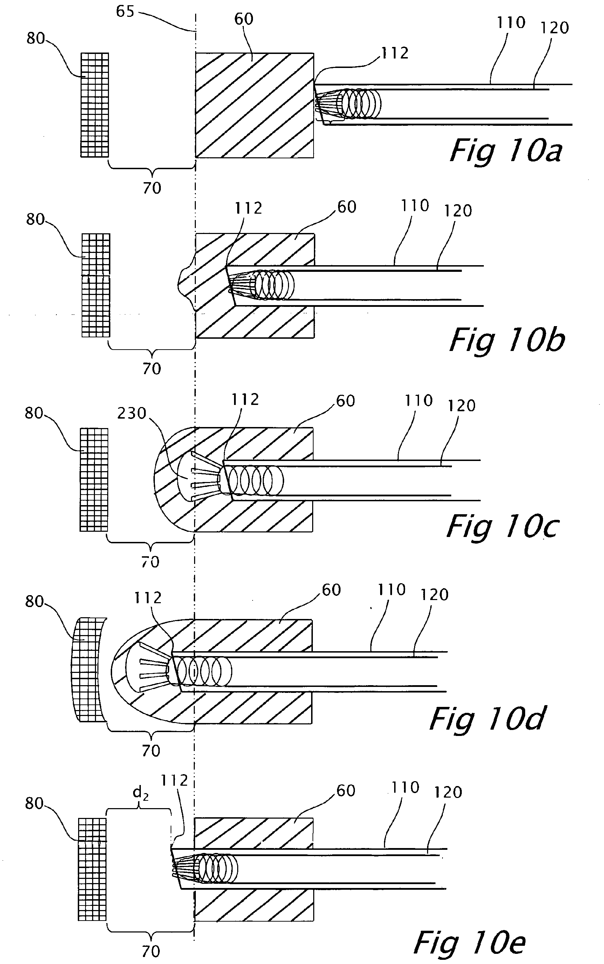

An aspect of the present invention is to provide a method of advancing introducer **110** accurately to a preconfigured in-vivo location, for example, into Epidural space **70,** in an Epidural anesthesia (block) procedure. Since, Overshooting of tip **112** of introducer **110** beyond Epidural space **70** may puncture Dura mater **80,** it would be advantageous to have a procedure that substantially reduce the risk of tip **112** of introducer **110** puncturing Dura mater **80.** It is the intention of the present invention to provide a spring-back method of erupting Ligamentum Flavum **60,** such that introducer **110** is inside Epidural space **70** at a distance *d₂* (see Fig. 10e) from Dura mater **80,** wherein distance *d₂* is substantially larger than distance *d₁* (see Fig. 2d): *d₂ >> d₁.* The spring-back method is designed for penetrating elastic tissues, and takes advantage of the elasticity of that tissue.

The principle idea behind the various spring-back methods is to bring tip **112** of introducer **110** to a special position beyond virtual line **65,** denoting the undistorted position of the wall of Ligamentum Flavum **60** that is the internal wall of Epidural space **70;** keep introducer **110** spatially stationary; use expandable device **130** of anatomical-positioning apparatus **100** to further push elastic Ligamentum Flavum **60** by a preconfigured distance; contract expandable device **130** at once and return expandable device **130** back inside introducer **110.** Thereby, the elasticity of Ligamentum Flavum **60** moves Ligamentum Flavum **60** towards the undistorted position **65,** rapidly. Thereby, spearingly meeting tip **112** of introducer **110,** on the way back, which cause Ligamentum Flavum **60** to rupture after one or more spring-back iterations.

In Figs. 10a-10e, a spring-back method, according to embodiments of the present invention, for puncturing, for example, Ligamentum Flavum **60** using, for example, expandable device **230** of anatomical-positioning apparatus **200** (Figs. 5 and 6), is illustrated. The spring-back method is described through the Epidural block procedure, without limiting the scope of this invention.

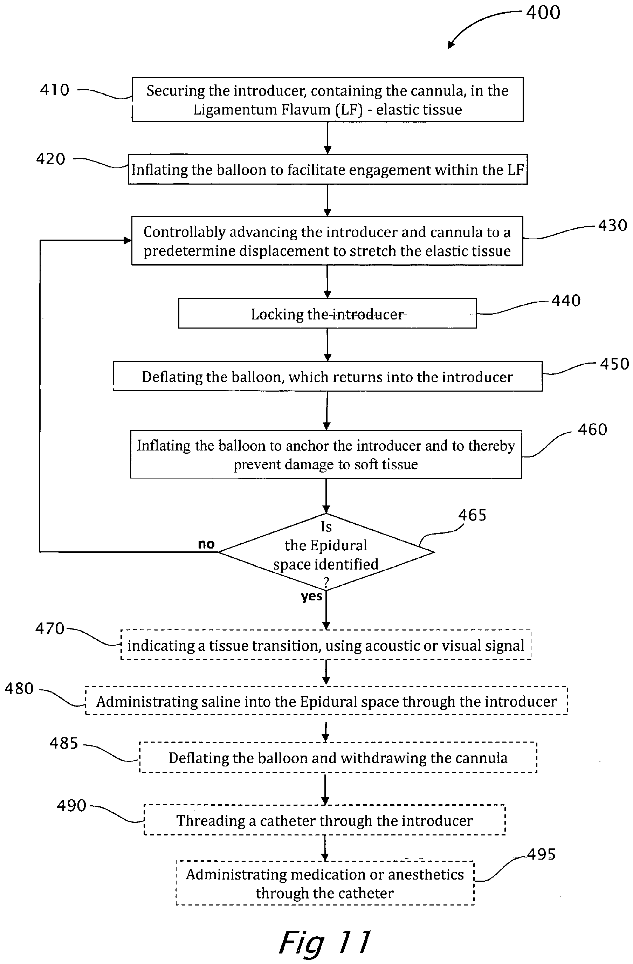

Reference is now made to Fig. 11, a schematic flow chart, showing an exemplary method **400** of safely advancing an introducer **110** into Epidural space **70** then further administrating medication or anesthetics through a catheter into Epidural space **70,** during an Epidural block procedure, according to embodiments of the present invention. Method **400** begins by administrating introducer **110** of anatomical-positioning apparatus **200** into a mammalian tissue, as shown in Fig. 10a. Method **400** proceeds with the following steps:
**Step 410:** securing introducer **110,** containing cannula **120,** in Ligamentum Flavum **60.** The operator of anatomical-positioning apparatus **200** secures introducer **110,** containing cannula **120,** in Ligamentum Flavum **60,** Ligamentum Flavum **60** being an elastic tissue (See Fig. 10b). The elastic tissue stretches slightly beyond the original, undistorted position **65.** Introducer **110** is advanced into Ligamentum Flavum **60** after departing from Interspinous Ligament **52.**
**Step 420:** inflating balloon **230** to facilitate engagement with the fibers of Ligamentum Flavum **60.**
   The operator activates expandable mechanism **150** and thereby, expandable device **230** is disposed out of introducer **110** and distal section **234** of balloon **230** expanded, typically by pressurized flow of gas (typically, air) or fluid, performed by expanding mechanism **150.** Distal section **234** of balloon **230** expands against flaps **256** of conical element **250,** causing flaps **256** to open outwardly, thereby protecting balloon **230** from being punctured by tip **112** of introducer **110** (Fig, 10c).
**Step 430:** controllably advancing introducer **110** and cannula **120** to a predetermine displacement to stretch the elastic tissue (Ligamentum Flavum **60**).
   The operator advances introducer **110** together with cannula **120** and expandable device **230** using introducer-incremental-advancing-mechanism **190** and cannula-incremental-advancing-mechanism **192** respectively, to a predetermined displacement (See Fig. 10d), while expandable device **230** is steadily expanded. Thereby, elastic tissue stretches extensively beyond the original, undistorted position **65** of the elastic tissue wall.
**Step 440:** locking introducer **110.**
   Locking introducer **110** in position, using introducer-incremental-advancing-mechanism **190.**
**Step 450:** deflating balloon **230,** which returns into introducer **110.**
   The operator rapidly deflates expandable device **230** to facilitate a rapid return of balloon **230** into introducer **110.** In a preferred embodiment, conical element **250** and spring **240** facilitate the rapid return on balloon **230** into introducer **110.** Ligamentum Flavum **60** rapidly returns to the undistorted position, towards and possibly over introducer **110.** If tip **112** of introducer **110** is disposed beyond the original, undistorted position **65** of Ligamentum Flavum **60,** introducer **110** penetrates Ligamentum Flavum **60** at a predetermined location.
**Step 460:** inflating balloon **230** to anchor introducer **110** and to thereby prevent damage to soft tissue.
   Balloon **230** is inflated to anchor introducer **110** and to thereby prevent introducer **110** from advancing forward towards soft tissues, thus protecting them. For example, if introducer is inside Epidural space, there is a need for protecting Dura mater **80** or other anatomical structures inside Epidural space such as blood vessels, from being punctured by introducer **110.**
**Step 465:** check if Epidural space **70** identified.
   Follow the procedure, such as method **300,** to identify the type of tissue. If the fat tissue of Epidural space **70** is not identified, but rather that Ligamentum Flavum **60** is still identified, go to step **430.**
   Else, optionally, proceed with the Epidural block procedure.
**Step 470:** indicating a tissue transition, using acoustic or visual signal.
   Indicator **180** indicates a tissue transition event (from Ligamentum Flavum **60** to Epidural space **70**), using an acoustic, visual or any other indicatory signal.
**Step 480:** administrating saline into Epidural space **70** through introducer **110**.
   Administrate saline into Epidural space **70** through introducer **110** to thereby locally enlarge Epidural space **70** to facilitate insertion of a catheter.
**Step 485:** deflating balloon **230** and withdrawing cannula **120.**
   Deflate balloon **230** and withdraw cannula **120** to facilitate insertion of a catheter.
**Step 490:** threading a catheter through introducer **110.**
   A catheter is inserted to Epidural space **70** through introducer **110.**
**Step 495:** administrating medication or anesthetics through the catheter.
   Administrate medication or anesthetics through the catheter.

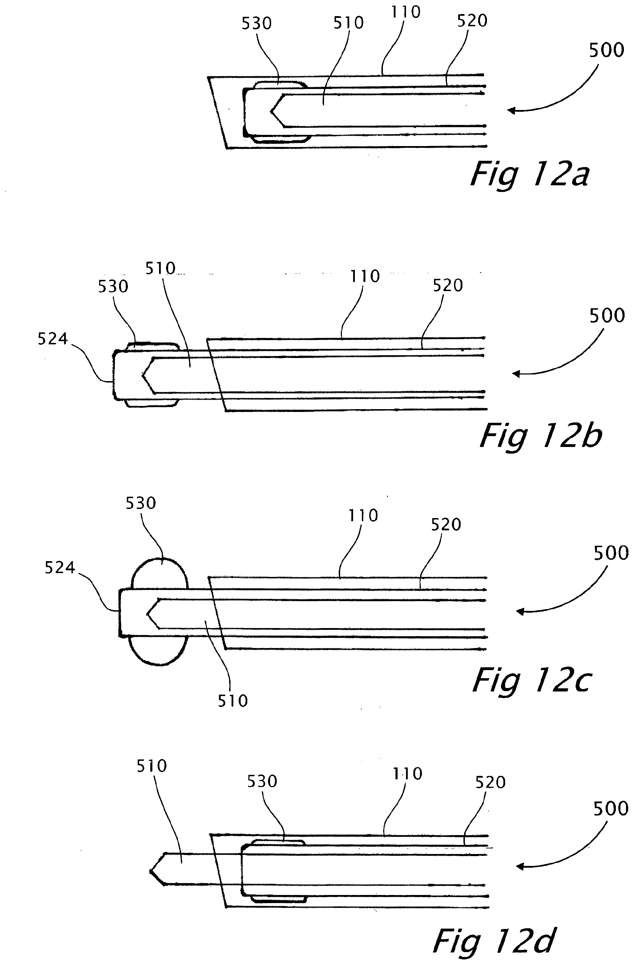

In Figs. 12a-12d, another anatomical-positioning apparatus **500** facilitating a spring-back method, according to other embodiments of the present invention, for puncturing, for example, Ligamentum Flavum **60** using, for example, expandable device **530** of anatomical-positioning apparatus **500,** is illustrated. The spring-back method is described through the Epidural block procedure, without limiting the scope of this invention. Anatomical-positioning apparatus **500** includes a cannula **520,** typically a double lumen cannula, and a thin-needle **510** facilitated to incrementally advance with respect to cannula **520.** The expandable device includes a balloon **530** disposed on the external circumference of cannula **520,** proximal to distal end **524** of cannula **520.**

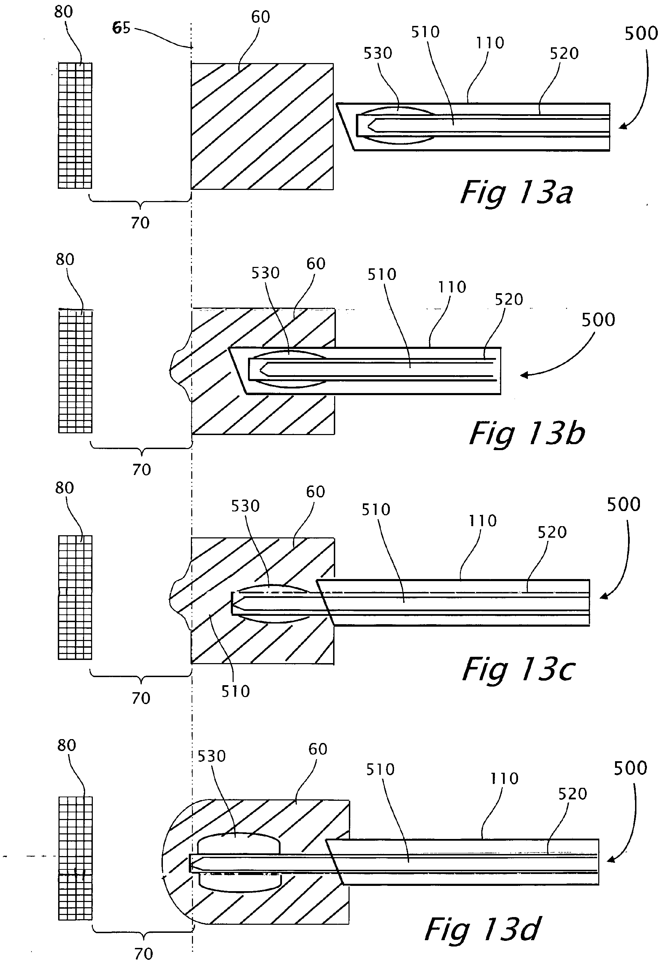

Figs. 13a-13g illustrate cross sectional views of the distal portion of anatomical-positioning apparatus **500,** showing the stages of a spring-back method of safe penetration of an elastic tissue, for example, Ligamentum Flavum **60** using, for example, expandable device **530** of anatomical-positioning apparatus **500,** is illustrated.

In this spring-back method, when the elasticity of Ligamentum Flavum **60** rapidly moves Ligamentum Flavum **60** back towards the undistorted position **65,** Ligamentum Flavum **60** is spearingly meeting the tip of thin-needle **510,** rather than tip **112** of introducer **110.** Eventually, thin-needle **510** causes Ligamentum Flavum **60** to rupture after one or more spring-back iterations.

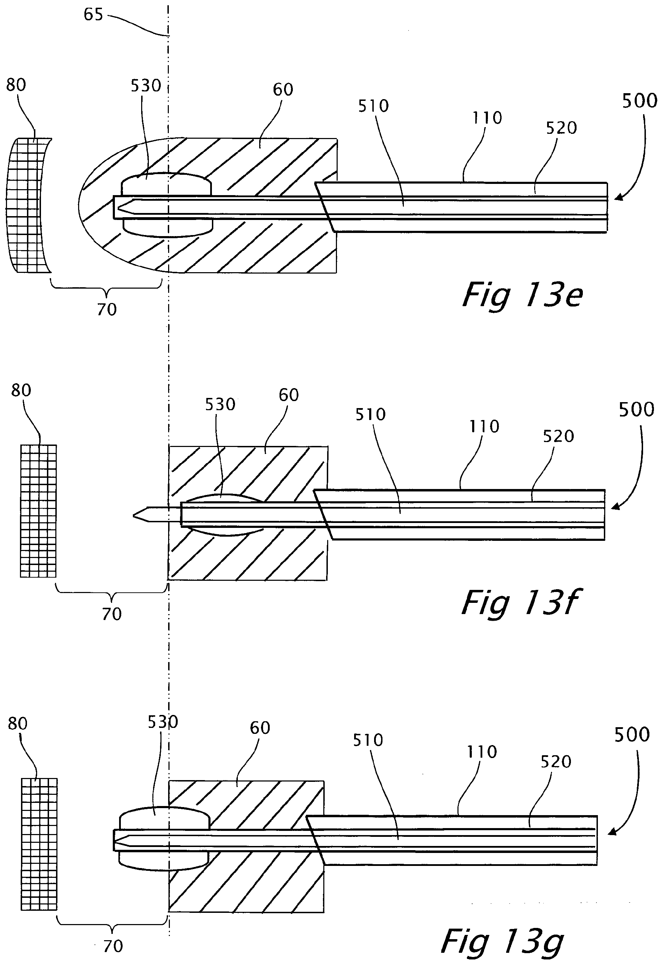

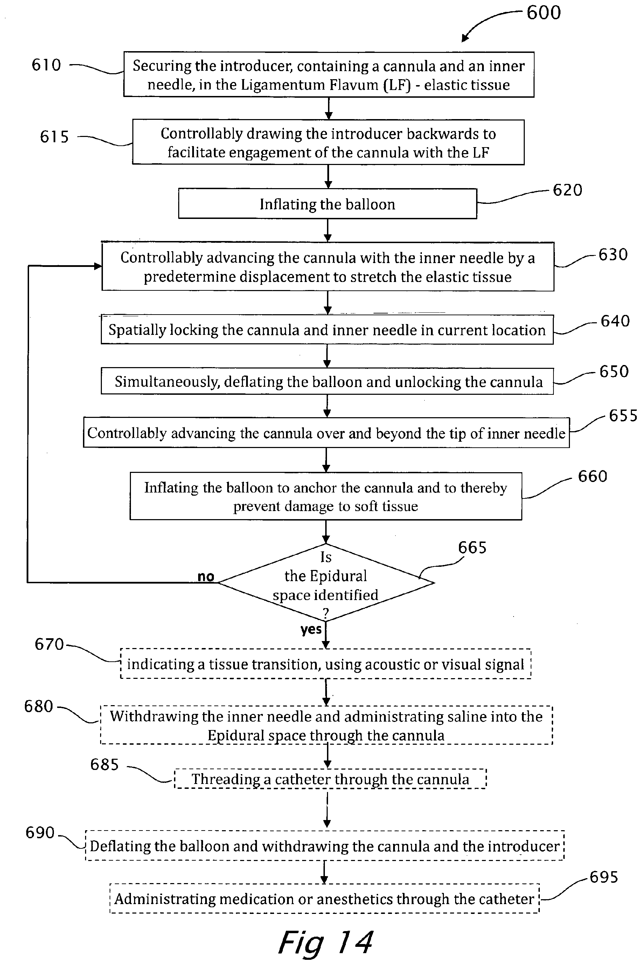

Reference is now made to Fig. 14, a schematic flow chart, showing an exemplary method **600** of safely advancing an inner needle **510** and a cannula **520** into Epidural space **70** then further administrating medication or anesthetics through the catheter into Epidural space **70,** during an Epidural block procedure, according to embodiments of the present invention. Method **600** begins by administrating introducer **110** of anatomical-positioning apparatus **500** into a mammalian tissue (See Fig. 13a). Method **600** proceeds with the following steps:
**Step 610:** securing introducer **110,** containing cannula **520** and inner needle **510,** in Ligamentum Flavum **60.**
   The operator of anatomical-positioning apparatus **500** secures introducer **110,** containing cannula **520** and inner needle **510,** in Ligamentum Flavum **60,** Ligamentum Flavum **60** being an elastic tissue (See Fig. 13b). The elastic tissue stretches slightly beyond the original, undistorted position **65.** Introducer **110** is advanced into Ligamentum Flavum **60** after departing from Interspinous Ligament **52.**
**Step 615:** Controllably drawing introducer **110** backwards, with respect to cannula **520.**
   The operator draws introducer **110** backwards, with respect to cannula **520** (see Fig. 13c), to facilitate engagement of cannula **520** and balloon **530,** attached thereto, with the fibers of Ligamentum Flavum **60.**
**Step 620:** inflating balloon **530.**
   The operator activates expandable mechanism **150** and thereby, balloon **530** is expanded (see Fig. 13d), typically by pressurized flow of gas (typically, air) or fluid, performed by expanding mechanism **150.** Balloon **530** expands against the fibers of Ligamentum Flavum **60.**
**Step 630:** controllably advancing cannula **520** and inner needle **510** by a predetermine displacement to stretch the elastic tissue (Ligamentum Flavum **60**).
   The operator advances cannula **520** (together with the expanded balloon **530**) and inner needle **510** by a predetermined displacement (see Fig. 13e). Thereby, elastic tissue stretches extensively beyond the original, undistorted position **65.**
**Step 640:** spatially locking cannula **520** and inner needle **510** in current location.
   Locking cannula **520** and inner needle **510** in position, using inner needle-incremental-advancing-mechanism and cannula-incremental-advancing-mechanism **192.**
**Step 650:** simultaneously, deflating balloon **530** and unlocking cannula **520.**
   Simultaneously, deflating balloon **530** and unlocking cannula **520**.
   Ligamentum Flavum **60** rapidly returns to the undistorted position, towards and possibly over inner needle **510.** If the tip of inner needle **510** is disposed beyond the original, undistorted position **65** of Ligamentum Flavum **60,** inner needle **510** penetrates Ligamentum Flavum **60** at a predetermined location (see Fig. 13f).
**Step 655:** controllably advancing cannula **520** over and beyond the tip of inner needle **510.**
   The operator advances cannula **520** over and beyond the tip of inner needle **510,** wherein cannula **520** serves both as a sheath for inner needle **510** to protect the immediate surroundings of inner needle **510** (see Fig. 13g) and as a pathway for the insertion of a catheter.
**Step 660:** inflating balloon **530** to anchor cannula **520** and to thereby prevent damage to soft tissue.
   Balloon **530** is inflated to anchor cannula **520** and to thereby prevent damage to soft tissue (see Fig. 13g). Should distal end **524** of cannula **520** have reached Epidural space **70,** prevent unwarranted motion of cannula **520** inside Epidural space **70.**
**Step 665:** check if Epidural space **70** identified.
   Follow the procedure, such as method **300,** to identify the type of tissue. If the fat tissue of Epidural space **70** is not identified, but rather that Ligamentum Flavum **60** is still identified, go to step **630.**
   Else, optionally, proceed with the Epidural block procedure.
**Step 670:** indicating a tissue transition, using acoustic or visual signal.
   Indicator **180** indicates a tissue transition event (from Ligamentum Flavum **60** to Epidural space **70**), using an acoustic, visual or any other indicatory signal.
**Step 680:** Withdrawing inner needle **510** and administrating saline into Epidural space **70** through cannula **520.**
   Withdraw inner needle **510 to** facilitate a pathway for administrating saline into Epidural space **70** through cannula **520** to thereby locally enlarge Epidural space **70** to facilitate insertion of a catheter.
**Step 685:** Threading a catheter through cannula **520.**
   A catheter'is inserted to Epidural space **70** through cannula **520**.
**Step 690:** deflating balloon **530** and withdrawing cannula **520** and introducer **110.**
   Deflating balloon **530** and withdrawing cannula **520** and introducer **110,** so that only a catheter is placed inside Epidural space **70.**
**Step 695:** administrating medication or anesthetics through the catheter.
   Administrate medication or anesthetics through the catheter.

An aspect of the present invention is to provide an anatomical-positioning apparatus and method of use thereof, having an introducer with an openable tip.

The principle idea behind an introducer with an openable tip is to have a balloon trapped inside the openable tip. The openable tip of the introducer has a closed state and an opened state, wherein in the closed state the openable tip forms a sharp tip facilitating penetration in and through a tissue, and wherein in the opened state the openable tip expands to forms a blunt surface, anchoring the introducer in position.

A preconfigured pressure is maintained inside the balloon trapped inside the openable tip. As long as introducer with a openable tip is situated inside a tissue, such as Ligamentum Flavum **60,** wherein the pressure applied on the openable tip by the tissue exceeds the preconfigured pressure inside the balloon, the openable tip stays in a closed state. Once the openable tip of the introducer moves into a softer tissue, such as in Epidural space **70,** wherein the surrounding pressure, due to tissue properties, is significantly lower than the internal pressure inside the balloon, the balloon expands rapidly. Thereby, the expanding balloon causes the openable tip of the introducer to open and anchor the openable tip of the introducer inside Epidural space **70,** wherein the balloon protects tissues and anatomical structures ahead of it.

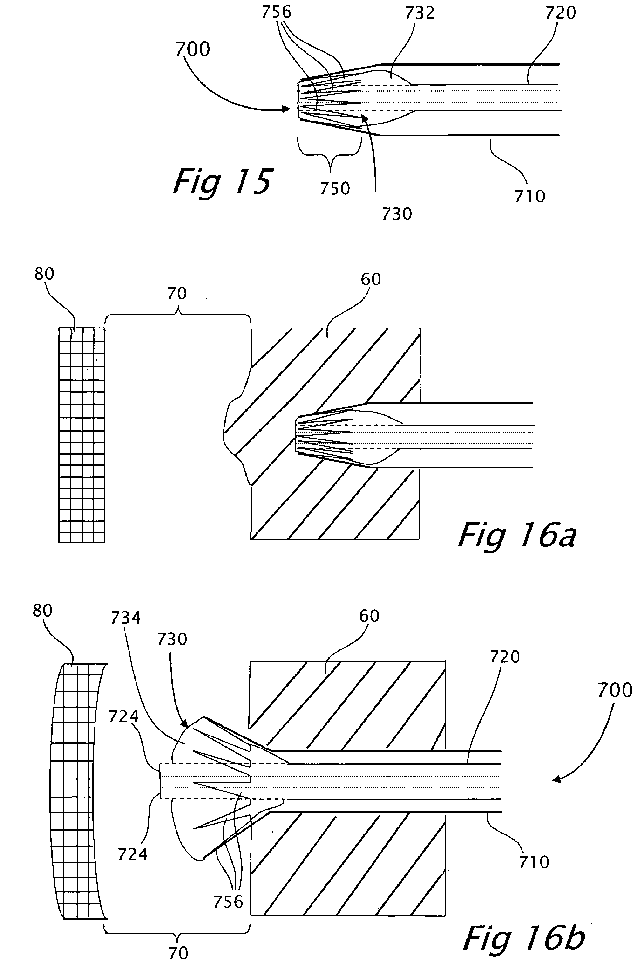

Figs. 15, 16a and 16b, illustrate the distal section of an anatomical-positioning apparatus **700,** according to variations of the present invention, includes an introducer **710** having an openable tip **750,** for puncturing a tissue, for example, Ligamentum Flavum **60.** Anatomical-positioning apparatus **700** is described through the Epidural block procedure, without limiting the scope of this invention. Fig. 15 illustrates openable tip **750** in a closed state. Fig. 16a illustrates openable tip **750** in a closed state penetrates through a tissue, such as Ligamentum Flavum **60.** Fig. 16b illustrates openable tip **750** in an opened state after penetrating into a softer tissue, such as Epidural space **70.**

Anatomical-positioning apparatus **700** further includes a cannula **720** having an expandable device **730,** typically a balloon, wherein proximal end **732** of balloon **730** is securely attached to the external circumference of cannula **720,** proximal to distal end **724** of cannula **720.** Openable tip **750** includes a preconfigured number of elastic flaps **756** that are separated by a slot cut into the distal section of flexible tip **750.** Typically, flaps **756** have a pointing tip and can operatively hingedly bend or deflect outwards as a result of a preconfigured pressure being built inside balloon **732,** when the pressure external to flaps **756** is lower than the pressure inside balloon **732.** Otherwise, balloon **730** stays trapped inside openable tip **750** of introducer **710** and thereby, openable tip **750** can be advanced through tissue.

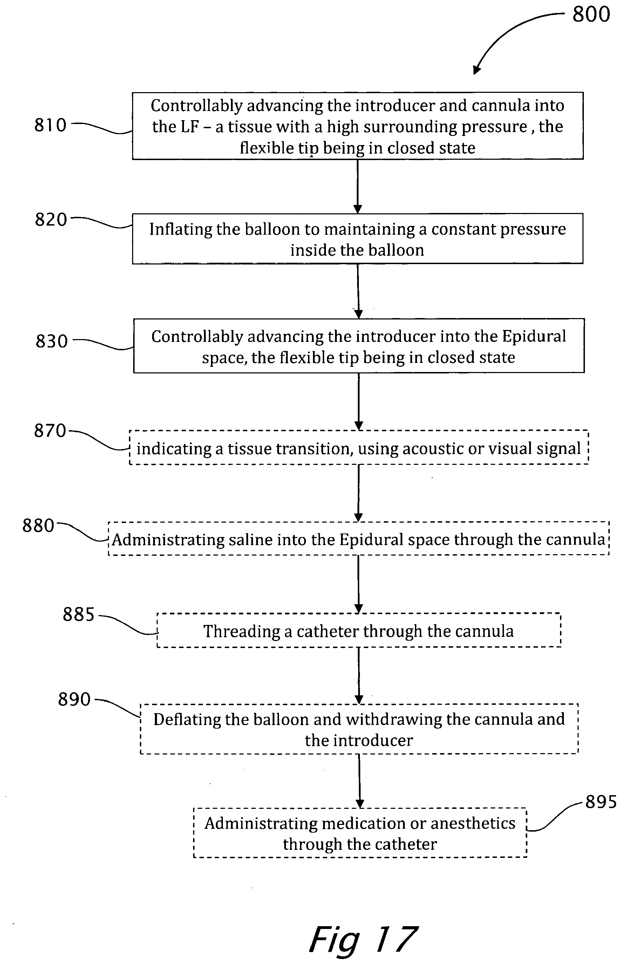

Reference is now made to Fig. 17, a schematic flow chart, showing an exemplary method **800** of administrate medication or anesthetics through the catheter into Epidural space **70,** during an Epidural block procedure, according to embodiments of the present invention. Method **800** begins by administrating introducer **710** of anatomical-positioning apparatus **700** into a mammalian tissue wherein openable tip **750** of introducer **710** is in closed state. Method **800** proceeds with the following steps:
**Step 810:** controllably advancing introducer **710,** containing cannula **720,** into and inside an elastic tissue.
   The operator advances introducer **710,** containing cannula **720,** into and inside a soft tissue with high surrounding pressure, such as Ligamentum Flavum **60.** Openable tip **750** is in a closed state.
**Step 820:** inflating balloon **730** to maintaining a constant pressure inside balloon **730.**
   The operator activates an expandable mechanism **150** to inflate balloon **730** and to thereby, maintaining a constant preconfigured pressure inside balloon **730.** Situated inside a soft tissue, having fibers that exert pressure on the external sides of flaps **756** the pressure being greater than the pressure inside balloon **730,** openable tip **750** remain in a closed state.
**Step 830:** controllably advancing introducer **710** and cannula **720** into Epidural space **70,** the openable tip being in closed state.
   The operator further advances introducer **710** and cannula **720,** openable tip **750** remaining in closed state. Once openable tip **750** of introducer **710** enters Epidural space **70,** the external pressure exerted on the external sides of flaps **756** drops substantially, bellow the preconfigured pressure inside balloon **730.** As a result, openable tip **750** opens up into an opened state as balloon **730** expands inside Epidural space **70,** thus protecting soft tissues and structures ahead of balloon **730.**
**Step 870:** indicating a tissue transition, using acoustic or visual signal.
   The indicator of anatomical-positioning apparatus **700** indicates a tissue transition event (from Ligamentum Flavum **60** to Epidural space **70**), using an acoustic, visual or any other indicatory signal.
**Step 880:** administrating saline into Epidural space **70** through cannula **720.**
   Administrate saline into Epidural space **70** through cannula **720** to thereby locally enlarge Epidural space **70** to facilitate insertion of a catheter.
**Step 885:** threading a catheter through cannula **720.**
   A catheter is inserted to Epidural space **70** through cannula **720.**
**Step 890:** deflating balloon **730** and withdrawing introducer **710** and cannula **720.**
   Deflate balloon **730** and withdrawing introducer **710** and cannula **720.**
**Step 895:** administrating medication or anesthetics through the catheter.
   Administrate medication or anesthetics through the catheter.

## Claims

1. An anatomical-positioning apparatus (100) for acquiring mechanical data from a mammalian tissue to facilitate determining a type of the mammalian tissue and determining transitions between different mammalian tissues and cavities, comprising:
a cannula (120) having a tip (124) at a distal section thereof and an external circumference that is proximal to the tip;
an expandable device (130), securely attached to the tip or on the external circumference of the cannula; the expandable device comprising at least one of: a balloon, a membrane, a diaphragm, a spring and a flexible device, wherein the expandable device has a contracted form size when in a contracted state and an expanded form size of preconfigured volume when in an expanded state;
an introducer (110) having a longitudinal axis and a distal end, the introducer arranged to penetrate and advance through the mammalian tissue and to facilitate an introduction of the expandable device in a contracted state into the mammalian tissue;
an expanding mechanism (150) arranged to controllably expand and contract the expandable device within the mammalian tissue to displace the tissue;
an advancement mechanism (192) arranged to incrementally and controllably advance at least one of: the introducer, the cannula, the expandable device, and a combination thereof;
a sensor (170) arranged to measure physical parameters associated with the expandable device and correlated with a mechanical response of the tissue to the displacement by the expandable device; and
a processor (160) arranged to record and analyze the measurements and determine therefrom the type of the mammalian tissue and the transitions between different mammalian tissues and cavities;
wherein the advancement mechanism and the expanding mechanism are configured to:
introduce the expandable device into the mammalian tissue, the introducer being advanced incrementally through the mammalian tissue either together with the cannula and the expandable device, or incrementally with respect to the cannula and the expandable device;
expand the expandable device by the expanding mechanism outside the tip of the cannula from said contracted form size to said expanded form size of preconfigured volume to exert pressure onto portions of the mammalian tissue to displace the mammalian tissue from an undistorted position to a distorted position; and,
said sensor is arranged to measure one or more measurements selected from a group of measurements consisting of the pressure inside the expandable device, an external force applied on the expandable device by the mammalian tissue, and the spatial pressure or spatial force applied on the expandable device by the mammalian tissue, while the expandable device exerts pressure onto portions of the mammalian tissue.

2. The anatomical-positioning apparatus as in claim 1, wherein the expandable device protects the mammalian tissue or an anatomical structure ahead of the mammalian tissue, from being punctured by the introducer of the expandable device.

3. The anatomical-positioning apparatus as in claim 1, wherein the expandable device expands and contracts under fluid or gas pressure provided by the expanding-mechanism through the cannula.

4. The anatomical-positioning apparatus as in claim 1, wherein the sensor comprises at least one of: a pressure sensor, a strain gauge, a force sensor, a tactile sensor, a displacement sensor, a volume sensor, a flow sensor and a piezoelectric transducer.

5. The anatomical-positioning apparatus as in claim 1, wherein:
the introducer is an openable introducer having an openable sharp tip that has a closed state and an open state;
the expandable device is trapped inside the openable sharp tip in the closed state and is configured, when the expandable device is loaded by a preconfigured expanding force and the sharp tip is situated in a high density tissue, to maintain the openable sharp tip in the closed state, wherein the closed state facilitates advancement of the openable introducer through the mammalian tissue; and
the expandable device is configured, when the expandable device is loaded by a preconfigured expanding force and the openable sharp tip enters a low density tissue or cavity, to facilitate an opening of the openable sharp tip, wherein the open state is configured to prevent advancement of the openable introducer through the mammalian tissue.

6. The anatomical-positioning apparatus as in claim 1, wherein the introducer is a hollow elongated member that is selected from a group of introducers consisting of a veress needle, an epidural needle, a biopsy needle, a trocar, a cannula, a catheter, a Tuohy type needle, a surgical instrument or any other sharp tool; wherein the cannula and the expandable device are positioned within the bore of the introducer, while the introducer facilitates the insertion of the cannula and the expandable device into the mammalian tissue.

7. The anatomical-positioning apparatus as in claim 3, wherein the cannula comprises a double lumen having a first and a second lumen, the first lumen facilitating a pathway for the fluid or gas into the expandable device, and the second lumen facilitating a pathway for at least one of: a thin needle, a catheter, an optical fiber, a miniature camera, an electrode, a guidewire, a surgical instrument and a sharp object.

8. The anatomical-positioning apparatus as in claim 3, wherein the cannula comprises a single lumen configured to facilitate a pathway for the fluid or gas into the expandable device and a pathway for an optical fiber or a miniature camera, to thereby facilitate visualization of the mammalian tissue through the expandable device, the expandable device being in contact with the mammalian tissue.

9. The anatomical-positioning apparatus as in claim 1, wherein the processor is arranged to derive at least one of: a volume-pressure work performed by the expandable device, a volume-pressure profile of the expandable device, a work being done by the expandable device and a displacement-force profile of the expandable device.

10. The anatomical-positioning apparatus as in claim 1, wherein the introducer is an elongated member that is selected from a group of introducers consisting of a thin needle, a veress needle, an epidural needle, a biopsy needle, a trocar, a cannula, a catheter, a Tuohy type needle, a guidewire, a surgical instrument or any other sharp tool; wherein the introducer is positioned within the lumen of the cannula, wherein the expandable device is securely attached to the external circumference of the cannula, proximal to the tip of the cannula, while the introducer facilitates the insertion of the cannula and the expandable device into the mammalian tissue.

11. The anatomical-positioning apparatus as in claim 1, arranged to measure the physical parameters during at least one of: while the mammalian tissue is being displaced by expanding the expandable device, while the mammalian tissue returns to its relaxed position by contracting the expandable device, and while the mammalian tissue is being displaced by maintaining a preconfigured pressure level or a preconfigured volume level of the expandable device.

## Patentansprüche

1. Anatomische Positioniervorrichtung (100) zum Erfassen mechanischer Daten von einem Säugetiergewebe, um ein Bestimmen einer Art des Säugetiergewebes und ein Bestimmen von Übergängen zwischen unterschiedlichen Säugetiergeweben und -hohlräumen zu ermöglichen, umfassend:
eine Kanüle (120) mit einer Spitze (124) an einem distalen Abschnitt davon und einem proximal zur Spitze verlaufenden Außenumfang;
eine aufweitbare Vorrichtung (130), die sicher an der Spitze oder dem Außenumfang der Kanüle befestigt ist; wobei die aufweitbare Vorrichtung mindestens eins von einem Ballon, einer Membran, einem Diaphragma, einer Feder und einer flexiblen Vorrichtung umfasst, wobei die aufweitbare Vorrichtung eine zusammengezogene Formgröße hat, wenn sie in einem zusammengezogenen Zustand ist, und eine aufgeweitete Formgröße eines vorab konfigurierten Volumens hat, wenn sie in einem aufgeweiteten Zustand ist;
ein Einführmittel (110) mit einer Längsachse und einem distalen Ende, wobei das Einführmittel dazu angeordnet ist, in Säugetiergewebe einzudringen und sich darin vorwärts zu bewegen, um das Einführen der aufweitbaren Vorrichtung in einem zusammengezogenen Zustand in das Säugetiergewebe zu ermöglichen;
einen Aufweitungsmechanismus (150), der angeordnet ist, um die aufweitbare Vorrichtung innerhalb des Säugetiergewebes steuerbar aufzuweiten und zusammenzuziehen, um das Gewebe zu dislozieren;
einen Vorwärtsbewegungsmechanismus (192), der angeordnet ist, um mindestens eines der Folgenden inkrementell und steuerbar vorwärts zu bewegen: das Einführmittel, die Kanüle, die aufweitbare Vorrichtung und eine Kombination davon;
einen Sensor (170), der angeordnet ist, um physische, mit der aufweitbaren Vorrichtung assoziierte Parameter zu messen und mit einer mechanischen Gewebereaktion auf die Verdrängung des Gewebes durch die aufweitbare Vorrichtung korreliert ist; und
einen Prozessor (160), der angeordnet ist, um die Messungen aufzuzeichnen und zu analysieren und darauf aufbauend die Art des Säugetiergewebes und die Übergänge zwischen verschiedenen Säugertiergeweben und -hohlräumen zu bestimmen;
wobei der Vorwärtsbewegungs- und der Aufweitungsmechanismus folgenderweise konfiguriert sind:
Zum Einführen der aufweitbaren Vorrichtung in das Säugetiergewebe, wobei das Einführmittel inkrementell durch das Säugetiergewebe vorwärtsbewegt wird, sei dies zusammen mit der Kanüle und der aufweitbaren Vorrichtung oder inkrementell in Bezug auf die Kanüle und die aufweitbare Vorrichtung;
Aufweiten der aufweitbaren Vorrichtung durch den Aufweitungsmechanismus außerhalb der Spitze der Kanüle von der zusammengezogenen Formgröße zur aufgeweiteten Formgröße eines vorab konfigurierten Volumens, um auf Abschnitte des Säugetiergewebes Druck auszuüben, um das Säugetiergewebe von einer entzerrten in eine verzerrte Position zu dislozieren; und
der Sensor wird angeordnet, um eine oder mehrere Messungen, ausgewählt von einer Gruppe von Messungen zu nehmen, bestehend aus dem Druck innerhalb der aufweitbaren Vorrichtung, einer extern von dem Säugetiergewebe auf die aufweitbare Vorrichtung ausgeübten Kraft und dem räumlichen Druck oder der räumlichen Kraft, der bzw. die von dem Säugetiergewebe auf die aufweitbare Vorrichtung ausgeübt wird, während die aufweitbare Vorrichtung auf Abschnitte des Säugetiergewebes Druck ausübt.

2. Anatomische Positioniervorrichtung nach Anspruch 1, wobei die aufweitbare Vorrichtung das Säugetiergewebe oder eine dem Säugetiergewebe vorgelagerte anatomische Struktur davor bewahrt, dass sie von dem Einführmittel der aufweitbaren Vorrichtung durchstoßen wird.

3. Anatomische Positioniervorrichtung nach Anspruch 1, wobei sich das aufweitbare Gerät unter Einwirkung von Fluid- oder Gasdruck, der durch den Aufweitungsmechanismus durch die Kanüle bereitgestellt wird, aufweitet und zusammenzieht.

4. Anatomische Positioniervorrichtung nach Anspruch 1, wobei der Sensor mindestens eines von Folgendem umfasst: einen Drucksensor, einen Dehnungsmesstreifen, einen Kraftsensor, einen Berührungssensor, einen Wegsensor, einen Volumensensor, einen Strömungssensor und einen piezoelektrischen Wandler.

5. Anatomische Positioniervorrichtung nach Anspruch 1, wobei
das Einführmittel ein öffnungsfähiges Einführmittel mit einer öffnungsfähigen, scharfen Spitze ist, das einen geschlossenen und einen geöffneten Zustand hat;
die aufweitbare Vorrichtung im geschlossenen Zustand innerhalb der öffnungsfähigen, scharfen Spitze eingeschlossen und dazu konfiguriert ist, wenn die aufweitbare Vorrichtung von einer vorab konfigurierten Aufweitungskraft geladen wird und die scharfe Spitze sich in einem hochdichten Gewebe befindet, die öffnungsfähige scharfe Spitze im geschlossenen Zustand zu halten, wobei der geschlossene Zustand ein Vorwärtsbewegen des öffnungsfähigen Einführmittels durch das Säugetiergewebe ermöglicht; und
die aufweitbare Vorrichtung dazu konfiguriert ist, wenn die aufweitbare Vorrichtung durch eine vorab konfigurierte Aufweitungskraft geladen wird und die öffnungsfähige, scharfe Spitze in ein niederdichtes Gewebe oder einen Hohlraum eintritt, ein Öffnen der öffnungsfähigen, scharfen Spitze zu ermöglichen, wobei der offene Zustand dazu konfiguriert ist, ein Vorwärtsbewegen des öffnungsfähigen Einführmittels durch das Säugetiergewebe zu verhindern.

6. Anatomische Positioniervorrichtung nach Anspruch 1, wobei das Einführmittel ein hohles, längliches Element ist, das von einer Gruppe bestehend aus den folgenden Einführmitteln ausgewählt ist: einer Veress-Nadel, einer Epiduralnadel, einer Biopsienadel, einem Trokar, einer Kanüle, einem Katheter, einer Tuohy-Nadel, einem chirurgischen Instrument oder einem beliebigen anderen scharfen Werkzeug; wobei die Kanüle und die aufweitbare Vorrichtung innerhalb des Bohrdurchmessers eines Einführmittels positioniert werden, während das Einführmittel das Einführen der Kanüle und der aufweitbaren Vorrichtung in das Säugetiergewebe ermöglicht.

7. Anatomische Positioniervorrichtung nach Anspruch 3, wobei die Kanüle, die ein erstes und ein zweites Lumen aufweist, zweilumig ist, wobei das erste Lumen einen Weg für das Fluid oder Gas in eine aufweitbare Vorrichtung hinein ermöglicht, und das zweite Lumen einen Weg für mindestens eines von einer dünnen Nadel, einem Katheter, einem Lichtwellenleiter, einer Miniaturkamera, einer Elektrode, einem Führungsdraht, einem chirurgischen Instrument und einem scharfen Objekt ermöglicht.

8. Anatomische Positioniervorrichtung nach Anspruch 3, wobei die Kanüle ein einzelnes Lumen umfasst, das dazu konfiguriert ist, einen Weg für das Fluid oder Gas in die aufweitbare Vorrichtung zu ermöglichen und einen Weg für einen Lichtwellenleiter oder eine Miniaturkamera, um damit die Darstellung des Säugetiergewebes durch die aufweitbare Vorrichtung zu ermöglichen, wobei die aufweitbare Vorrichtung mit dem Säugetiergewebe in Kontakt ist.

9. Anatomische Positioniervorrichtung nach Anspruch 1, wobei der Prozessor dazu angeordnet ist, mindestens eins von Folgenden abzuleiten: eine Druck-Volumen-Arbeit, die von der aufweitbaren Vorrichtung durchgeführt wird; ein Volumen-Druck-Profil von der aufweitbaren Vorrichtung; eine von der aufweitbaren Vorrichtung verrichtete Arbeit und ein Verdrängungs-Kraft-Profil der aufweitbaren Vorrichtung.

10. Anatomische Positioniervorrichtung nach Anspruch 1, wobei das Einführmittel ein längliches Element ist, das von einer Gruppe von Einführmitteln bestehend aus einer dünnen Nadel, einer Veress-Nadel, einer Epiduralnadel, einer Biopsienadel, einem Trokar, einer Kanüle, einem Katheter, einer Tuohy-Nadel, einem Führungsdraht, einem chirurgischen Instrument oder einem beliebigen anderen scharfen Werkzeug ausgewählt ist; wobei das Einführmittel innerhalb des Lumens der Kanüle positioniert wird, wobei die aufweitbare Vorrichtung sicher am Außenumfang der Kanüle proximal zur Spitze der Kanüle befestigt ist, während das Einführmittel das Einführen der Kanüle und der aufweitbaren Vorrichtung in das Säugetiergewebe ermöglicht.

11. Anatomische Positioniervorrichtung nach Anspruch 1, die angeordnet ist, um die physischen Parameter während mindestens einem von Folgenden zu messen: während das Säugetiergewebe durch Aufweiten der aufweitbaren Vorrichtung disloziert wird, während das Säugetiergewebe durch Zusammenziehen der aufweitbaren Vorrichtung in seine entspannte Position zurückkehrt und während das Säugetiergewebe durch Aufrechterhalten eines vorab konfigurierten Druckstands oder eines vorab konfigurierten Volumenstands der aufweitbaren Vorrichtung disloziert wird.

## Revendications

1. Appareil de positionnement anatomique (100) pour acquérir des données mécaniques à partir d'un tissu mammalien pour faciliter la détermination d'un type du tissu mammalien et la détermination de transitions entre différents tissus et cavités mammaliens, comprenant :
une canule (120) ayant une pointe (124) à une section distale de celle-ci et une circonférence externe qui est proximale par rapport à la pointe ;
un dispositif expansible (130), solidement fixé à la pointe ou sur la circonférence externe de la canule ; le dispositif expansible comprenant au moins l'un parmi : un ballonnet, une membrane, un diaphragme, un ressort et un dispositif flexible, dans lequel le dispositif expansible a une taille sous forme contractée lorsqu'il est dans un état contracté et une taille sous forme expansée de volume préconfiguré lorsqu'il est dans un état expansé ;
un introducteur (110) ayant un axe longitudinal et une extrémité distale, l'introducteur agencé pour pénétrer et avancer à travers le tissu mammalien et pour faciliter une introduction du dispositif expansible dans un état contracté dans le tissu mammalien ;
un mécanisme d'expansion (150) agencé pour expanser et contracter de manière contrôlée le dispositif expansible au sein du tissu mammalien pour déplacer le tissu ;
un mécanisme d'avancement (192) agencé pour faire avancer de manière incrémentielle et contrôlée au moins l'un parmi : l'introducteur, la canule, le dispositif expansible, et une combinaison de ceux-ci ;
un capteur (170) agencé pour mesurer des paramètres physiques associés au dispositif expansible et se corrélant à une réponse mécanique du tissu au déplacement par le dispositif expansible ; et
un processeur (160) agencé pour enregistrer et analyser les mesures et déterminer à partir de celles-ci le type du tissu mammalien et les transitions entre différents tissus et cavités mammaliens ;
dans lequel le mécanisme d'avancement et le mécanisme d'expansion sont configurés pour :
introduire le dispositif expansible dans le tissu mammalien, l'introducteur étant avancé de manière incrémentielle à travers le tissu mammalien soit conjointement avec la canule et le dispositif expansible, soit de manière incrémentielle par rapport à la canule et au dispositif expansible ;
expanser le dispositif expansible par le mécanisme d'expansion à l'extérieur de la pointe de la canule depuis ladite taille sous forme contractée jusqu'à ladite taille sous forme expansée de volume préconfiguré pour exercer une pression sur des parties du tissu mammalien pour déplacer le tissu mammalien d'une position non déformée à une position déformée ; et,
ledit capteur est agencé pour mesurer une ou plusieurs mesures choisies dans un groupe de mesures constituées de la pression à l'intérieur du dispositif expansible, une force externe appliquée sur le dispositif expansible par le tissu mammalien, et la pression spatiale ou force spatiale appliquée sur le dispositif expansible par le tissu mammalien, alors que le dispositif expansible exerce une pression sur des parties du tissu mammalien.

2. Appareil de positionnement anatomique selon la revendication 1, dans lequel le dispositif expansible protège le tissu mammalien ou une structure anatomique devant le tissu mammalien, d'une perforation par l'introducteur du dispositif expansible.

3. Appareil de positionnement anatomique selon la revendication 1, dans lequel le dispositif expansible s'expanse et se contracte sous une pression de fluide ou de gaz fournie par le mécanisme d'expansion à travers la canule.

4. Appareil de positionnement anatomique selon la revendication 1, dans lequel le capteur comprend au moins l'un parmi : un capteur de pression, une jauge de contrainte, un capteur de force, un capteur tactile, un capteur de déplacement, un capteur de volume, un capteur d'écoulement et un transducteur piézoélectrique.

5. Appareil de positionnement anatomique selon la revendication 1, dans lequel :
l'introducteur est un introducteur ouvrable ayant une pointe effilée ouvrable qui a un état fermé et un état ouvert ;
le dispositif expansible est piégé à l'intérieur de la pointe effilée ouvrable dans l'état fermé et est configuré, lorsque le dispositif expansible est chargé par une force d'expansion préconfigurée et que la pointe effilée se trouve dans un tissu à haute densité, pour maintenir la pointe effilée ouvrable dans l'état fermé, dans lequel l'état fermé facilite un avancement de l'introducteur ouvrable à travers le tissu mammalien ; et
le dispositif expansible est configuré, lorsque le dispositif expansible est chargé par une force d'expansion préconfigurée et que la pointe effilée ouvrable entre dans un tissu ou une cavité à faible densité, pour faciliter une ouverture de la pointe effilée ouvrable, dans lequel l'état ouvert est configuré pour empêcher un avancement de l'introducteur ouvrable à travers le tissu mammalien.

6. Appareil de positionnement anatomique selon la revendication 1, dans lequel l'introducteur est un élément allongé creux qui est choisi dans un groupe d'introducteur constitué d'une aiguille de Veress, une aiguille épidurale, une aiguille de biopsie, un trocart, une canule, un cathéter, une aiguille de type Tuohy, un instrument chirurgical ou n'importe quel autre outil effilé ; dans lequel la canule et le dispositif expansible sont positionnés au sein de l'alésage de l'introducteur, alors que l'introducteur facilite l'insertion de la canule et du dispositif expansible dans le tissu mammalien.

7. Appareil de positionnement anatomique selon la revendication 3, dans lequel la canule comprend une double lumière ayant une première et une deuxième lumière, la première lumière frayant un passage pour le fluide ou gaz dans le dispositif expansible, et la deuxième lumière frayant un passage pour au moins l'un parmi : une aiguille fine, un cathéter, une fibre optique, une caméra miniature, une électrode, un fil-guide, un instrument chirurgical et un objet effilé.

8. Appareil de positionnement anatomique selon la revendication 3, dans lequel la canule comprend une lumière unique configurée pour frayer un passage pour le fluide ou gaz dans le dispositif expansible et un passage pour une fibre optique ou une caméra miniature, pour faciliter de ce fait la visualisation du tissu mammalien à travers le dispositif expansible, le dispositif expansible étant en contact avec le tissu mammalien.

9. Appareil de positionnement anatomique selon la revendication 1, dans lequel le processeur est agencé pour dériver au moins l'un parmi : un travail volume-pression effectué par le dispositif expansible, un profil volume-pression du dispositif expansible, un travail étant réalisé par le dispositif expansible et un profil déplacement-force du dispositif expansible.

10. Appareil de positionnement anatomique selon la revendication 1, dans lequel l'introducteur est un élément allongé qui est choisi dans un groupe d'introducteurs constitué d'une aiguille fine, une aiguille de Veress, une aiguille épidurale, une aiguille de biopsie, un trocart, une canule, un cathéter, une aiguille de type Tuohy, un fil-guide, un instrument chirurgical ou n'importe quel autre outil effilé ; dans lequel l'introducteur est positionné au sein de la lumière de la canule, dans lequel le dispositif expansible est solidement fixé à la circonférence externe de la canule, proximal par rapport à la pointe de la canule, alors que l'introducteur facilite l'insertion de la canule et du dispositif expansible dans le tissu mammalien.

11. Appareil de positionnement anatomique selon la revendication 1, agencé pour mesurer les paramètres physiques pendant au moins l'un parmi : tandis que le tissu mammalien est en cours de déplacement par expansion du dispositif expansible, tandis que le tissu mammalien retourne à sa position relaxée par contraction du dispositif expansible, et tandis que le tissu mammalien est en cours de déplacement en maintenant un niveau de pression préconfiguré ou un niveau de volume préconfiguré du dispositif expansible.
